(19) Europäisches Patentamt
European Patent Office
Office européen des brevets

(11) Veröffentlichungsnummer: **0 093 084**
**B1**

# (12) EUROPÄISCHE PATENTSCHRIFT

(45) Veröffentlichungstag der Patentschrift: **12.03.86**

(21) Anmeldenummer: **83810166.5**

(22) Anmeldetag: **20.04.83**

(51) Int. Cl.⁴: **C 07 D 209/42,** C 07 D 401/12, C 07 D 405/12, A 61 K 31/40 // (C07D401/12, 209:42, 213:30),(C07D405/12, 209:42, 307:89)

(54) **1-Carboxyalkanoylindolin-2-carbonsäure-ester, Verfahren zu ihrer Herstellung, pharmazeutische Präparate enthaltend diese Verbindungen, sowie ihre therapeutische Verwendung.**

(30) Priorität: **26.04.82 US 371700**

(43) Veröffentlichungstag der Anmeldung:
**02.11.83 Patentblatt 83/44**

(45) Bekanntmachung des Hinweises auf die Patenterteilung:
**12.03.86 Patentblatt 86/11**

(84) Benannte Vertragsstaaten:
**AT BE CH DE FR GB IT LI LU NL SE**

(56) Entgegenhaltungen:
**EP - A - 0 024 852**
**EP - A - 0 050 850**
**US - A - 3 796 723**

(73) Patentinhaber: **CIBA-GEIGY AG, Klybeckstrasse 141, CH-4002 Basel (CH)**

(72) Erfinder: **Gruenfeld, Norbert, Dr., 19 Sparrow Circle, White Plains New York 10605 (US)**

**EP 0 093 084 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

In der US-Patentschrift 3796723 werden substituierte Indolin-2-carbonsäuren, deren Alkylester, Amide sowie Mono- und Dialkylamide offenbart, deren Ringstickstoffatom als Substituenten unter anderem einen Acylrest tragen kann. Ein solcher Acylrest trägt in keinem Fall seinerseits eine veresterte Carboxylgruppe. Die offenbarten Verbindungen werden als hypotensive, antipyretische und anti-Parkinson-Mittel beschrieben.

In der europäischen Patentanmeldung 24852 werden neben tricyclischen Verbindungen auch N-acylierte Indolin-2-carbonsäuren, deren Amide und Niederalkylester beschrieben, die als Inhibitoren des Angiotensin umwandelnden Enzyms wirken und als Antihypertensiva eingesetzt werden können. Diese weisen im Acylrest in α- oder β-Stellung obligatorisch einen über Sauerstoff, Schwefel oder Stickstoff gebundenen Substituenten auf.

Die vorliegende Erfindung betrifft neue 1-Carboxy(alkanoyl oder aralkanoyl)indolin-2-carbonsäureester der allgemeinen Formel I

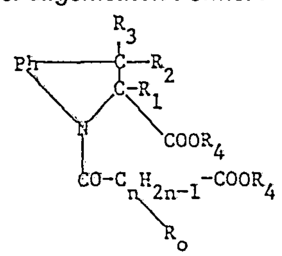
(I)

worin Ph unsubstituiertes 1,2-Phenylen oder durch einen bis drei, gleiche oder verschiedene Substitueten der Gruppe Niederalkyl, Niederalkoxy, Niederalkylendioxy, Hydroxy, Halogen oder Trifluormethyl substituiertes 1,2-Phenylen bedeutet, $R_o$ für Wasserstoff oder HPh steht, jedes der Symbole $R_1$, $R_2$ und $R_3$ Wasserstoff oder Niederalkyl bedeutet, n für eine ganze Zahl von 1 bis 10 steht; jedes der Symbole $R_4$ ist ein Substituent der Gruppe Aryl(nieder)alkyl, worin Aryl ein ggf. durch Niederalkyl, Niederalkoxy oder Halogen substituiertes Phenyl, oder Pyridyl bedeutet, Niederalkanoyloxy(nieder)alkyl, ggf. durch Niederalkyl, Niederalkoxy oder Halogen substituiertes Phthalidyl, (Hydroxy-, Niederalkanoyloxy- oder Niederalkoxy-)substituiertes (Nieder)alkoxymethyl, Bicycloalkyloxycarbonyl(nieder)alkyl, welches in der Bicycloalkylgruppe höchstens 10 Kohlenstoffatome aufweist und ggf. durch Niederalkyl substituiert ist, oder 1-(Niederalkoxycarbonyloxy)niederalkyl; oder eines der Symbole $R_4$ für Wasserstoff steht und das andere einen oben genannten Rest bedeutet, und ihre Salze, insbesondere ihre therapeutisch verwendbaren Salze, sowie Verfahren zu ihrer Herstellung, pharmazeutische Präparate enthaltend diese Verbindungen, sowie ihre therapeutische Verwendung.

Bevorzugt sind Verbindungen der Formel I, worin Ph unsubstituiertes 1,2-Phenylen, oder durch einen oder zwei, gleiche oder verschiedene Substituenten der Gruppe Niederalkyl, Niederalkoxy, Hydroxy oder Halogen oder durch ein Niederalkylendioxy oder Trifluormethyl substituiertes 1,2-Phenylen bedeutet, $R_o$ für Wasserstoff oder HPh steht; jedes der Symbole $R_1$, $R_2$ und $R_3$ Wasserstoff oder Niederalkyl bedeutet, n für eine ganze Zahl von 1 bis 10 steht; und $R_4$ die oben angegebene Bedeutung hat, und ihre Salze, insbesondere ihre therapeutisch verwendbaren Salze, in erster Linie die Alkalimetall-, Erdalkalimetall- und Ammoniumsalze der genannten Säuren.

Bevorzugt sind weiter solche Verbindungen der Formel I, worin Ph unsubstituiertes 1,2-Phenylen, oder durch Niederalkyl, Niederalkoxy, Niederalkylendioxy, Hydroxy, Halogen oder Trifluormethyl monosubstituiertes 1,2-Phenylen bedeutet, $R_o$ für Wasserstoff oder HPh steht, jedes der Symbole $R_1$, $R_2$ und $R_3$ Wasserstoff oder Niederalkyl bedeutet, n für die ganze Zahl von 2 bis 8 steht, und $R_4$ die oben angegebene Bedeutung hat, und ihre Salze, insbesondere ihre therapeutisch verwendbaren Salze.

Die 1,2-Phenylengruppe Ph und/oder die Phenylgruppe HPh sind vorzugsweise unsubstituiert oder monosubstituiert. Ihre Substituenten können durch die folgenden Gruppen illustriert werden: Niederalkyl, z. B. Methyl, Äthyl, n- oder iso-Propyl oder -Butyl; Niederalkoxy, z. B. Methoxy, Äthoxy, n- oder iso-Propoxy oder -Butoxy; Niederalkylendioxy, z. B. Methylendioxy, 1,1- oder 1,2-Äthylendioxy; Hydroxy; Halogen, z. B. Fluor, Chlor oder Brom; oder Trifluormethyl.

Die Gruppe Ph bedeutet vorzugsweise unsubstituiertes oder durch Chlor, Methoxy oder Methyl monosubstituiertes 1,2-Phenylen. Auch der Phenylrest HPh bedeutet vorzugsweise unsubstituiertes oder durch Chlor, Methoxy oder Methyl monosubstituiertes Phenyl.

Jedes der Symbole $R_1$, $R_2$ und $R_3$ bedeutet vorzugsweise Wasserstoff, aber auch Niederalkyl, vorzugsweise Methyl, oder eine andere, vorher genannte Niederalkylgruppe.

Der Ausdruck nieder definiert in den oben oder nachfolgend genannten organischen Resten oder Verbindungen solche mit höchstens 7, vorzugsweise 4, insbesondere 1 oder 2 Kohlenstoffatomen.

Die Alkylen- oder Aralkylengruppierung $C_nH_{2n-1}-R_o$ ist entweder geradkettig oder vorzugsweise verzweigt, und enthält vorteilhafterweise bis zu 8 Kohlenstoffatomen. So bedeutet sie, wenn $R_o$ Wasserstoff ist, z. B. Äthylen, 1,2- oder 1,3-Propylen, 2-Methyl-1,2- oder -1,3-propylen, 1,2-, 1,3-, 2,3- oder 1,4-Butylen, 1,2- 1,3-, 1,4-, 2,4- oder 1,5-Pentylen. Bedeutet $R_o$ Phenyl, so steht die oben genannte Gruppierung für ω-Phenyl-(1,2-, 1,3- oder 2,3-propylen, -butylen oder -pentylen, 1,3-, 2,3- oder 2,4-butylen, -pentylen oder -hexylen, oder 3,5-heptylen oder -octylen).

Die genannten Mono- oder Bisester von Indolin-2-carbonsäuren der Formel I, in welchen entweder eine oder die beiden Carboxygruppen verestert sind, sind vorzugsweise Aryl(nieder)alkylester, z. B. Benzyl-, (Methyl-, Methoxy-, Chlor)-substituierte Benzyl-, und Pyridylmethylester; Niederalkanoyloxy(nieder)alkylester, z. B. Pivaloyloxymethylester; 3-Phthalidyl- und (Methyl-, Methoxy-, Chlor-)substituierte 3-Phthalidylester, welche von entsprechenden 3-Hydroxyphthaliden abgeleitet sind, (Hydroxy-, Niederalkanoyloxy-, Niederalkoxy-)substituierte Niederalkoxymethylester, z. B. β-(Hydroxy-, Acetyloxy-, Methoxy-)äthoxymethylester; Bicycloalkyloxycarbonyl-(nieder)alkylester, z. B. solche von bicyclischen Monoterpenoidalkoholen, wie unsubstituierte oder Niederalkyl-substituierte Bicyclo-[2,2,1]-heptyloxycarbonyl(nieder)alkylester, vorzugsweise Bornyloxycarbonylmethylester, oder 1-(Niederalkoxycarbonyloxy)niederalkylester, z. B. 1-(Methoxy-, Äthoxy- oder Propoxycarbonyloxy)methyl, -äthyl oder -propylester.

Besonders bevorzugt sind die monofunktionellen Derivate von Dicarbonsäuren der Formel I, welche eine freie Indolin-2-carboxygruppe haben.

Salze sind vorzugsweise therapeutisch verwendbare Salze, z. B. Metall- oder Ammoniumsalze der genannten Säuren, insbesondere Alkalimetall- oder Erdalkalimetallsalze, z. B. Natrium-, Kalium-, Magnesium- oder Calciumsalze; in erster Linie leicht kristallisierende Ammoniumsalze. Diese werden abgeleitet von Ammoniak oder organischen Aminen, z. B. Mono-, Di- oder Trinieder(alkyl, cycloalkyl oder hydroxyalkyl)aminen, Niederalkylendiaminen oder (Hydroxyniederalkyl oder Arylniederalkyl)niederalkylammoniumbasen, z. B. Methylamin, Diäthylamin, Triäthylamin, Dicyclohexylamin, Triäthanolamine, Äthylendiamin, Tris-(hydroxymethyl)aminomethan oder Benzyltrimethylammoniumhydroxid. Die genannten basischen Ester bilden auch Säureadditionsalze. Diese werden vorzugsweise mit solchen Säuren, welche therapeutisch verwendbare Säureadditionssalze ergeben, hergestellt. Säuren, die therapeutisch verwendbare Säureadditionssalze ergeben, sind z. B. anorganische Säuren, wie Halogenwasserstoffsäuren, z. B. Chlorwasserstoff- oder Bromwasserstoffsäure, oder Schwefel-, Phosphor-, Salpeter- oder Perchlorsäure; aliphatische oder aromatische Carbon- oder Sulfonsäuren, z. B. Ameisen-, Essig-, Propion-, Bernstein-, Glykol-, Milch-, Äpfel-, Wein-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoe-, Nikotin-, Methansulfon-, Äthansulfon-, Hydroxyäthansulfon-, Äthylensulfon-, Halogenbenzolsulfon-, Toluolsulfon-, Naphthalinsulfon-, Sulfanil- oder Cyclohexylsulfaminsäure; oder die Ascorbinsäure.

Ausser den obigen, therapeutisch verwendbaren Salzen bilden auch ihre *prodrug*-Derivate, z. B. therapeutisch anwendbare Ester von Carbonsäuren der Erfindung, welche durch Solvolyse oder unter physiologischen Bedingungen in die genannten Carbonsäuren umgewandelt werden können, einen Gegenstand der Erfindung.

Solche Ester sind vorzugsweise, z. B. geradkettige oder verzweigte, in geeigneter Weise substituierte, Niederalkylester, z. B. Pivaloyloxymethyl-, Bornyloxycarbonylmethyl-, Pyridylmethylester und ähnliche, die gemäss an sich bekannten Methoden oder wie in den Beispielen beschrieben hergestellt werden können.

Die Verbindungen der Erfindung zeigen wertvolle pharmakologische Eigenschaften, in erster Linie hypotensive, antihypertensive und kardioaktive Wirkungen, welche sie unter anderem aufgrund ihrer Hemmwirkung auf das Enzym, welches das Angiotensin umwandelt, hervorrufen. Diese pharmakologischen Eigenschaften können durch *in vivo*- oder *in vitro*-Tierversuche, vorzugsweise an Säugetieren, wie Ratten, Katzen, Hunden oder ihren isolierten Organen, als Testobjekte nachgewiesen werden. Die Tiere können entweder normotensiv oder hypertensiv, z. B. genetisch hypertensive Ratten, oder renal hypertensive Ratten oder Hunde, und Hunde, denen Natrium entzogen worden ist, sein. Die genannten Verbindungen können ihnen enteral oder parenteral, vorzugsweise oral oder intravenös, z. B. durch Gelatinekapseln oder in Form von Stärke enthaltenden Suspensionen bzw. wässerigen Lösungen, verabreicht werden. Die verwendete Dosis kann in einem Bereich von ungefähr zwischen 0,01 und 100, vorzugsweise ungefähr 0,1 und 50, insbesondere ungefähr 0,1 und 25 mg/kg/d liegen.

Die *in vivo* blutdrucksenkende Wirkung wird entweder direkt mit einem Katheter, der z. B. in die femurale Arterie eines Hundes eingeführt ist, oder indirekt durch Sphygmomanometrie am Rattenschwanz, und mit einem Übertragungsinstrument registriert. Der Blutdruck wird vor und nach der Verabreichung des Wirkstoffes bestimmt. So sind z. B. die repräsentativen Verbindungen dieser Erfindung, welche in den Beispielen illustriert werden, in hypertensiven Ratten oder Hunden bereits bei einer Verabreichung in niedrigen oralen Dosen von 50 mg/kg/d oder darunter, sehr wirksam.

Die neuen Verbindungen zeigen auch eine Hemmwirkung auf das Ansprechen des Blutdrucks von normotensiven Ratten auf Angiotensin I. Das Enzym Renin verursacht unter normalen Verhältnissen eine spezifische Hydrolyse des zirkulierenden Renin-Substrat-Proteins. Diese Hydrolyse ergibt das Angiotensin I, welches durch die Einwirkung des genannten Enzyms, welches das Angiotensin umwandelt, in das stark gefässverengernde Angiotensin II weiter hydrolysiert wird. Durch Hemmung des genannten Enzyms wird die Entstehung des Angiotensins II aus I verhindert. Diese Hemmung dämpft daher jedes Ansprechen des Blutdrucks nach einer Angiotensin-I-Attacke.

Der entsprechende *in vivo*-Test wird mit männlichen, normotensiven Ratten, welche mit 100-120 mg/kg intraperitoneal verabreichtem Natriumsalz des Äthyl-(1-methylpropyl)malonylthioharnstoffs narkotisiert sind, vorgenommen. Eine femurale Arterie und eine Wadenvene werden mit je einer Hohlnadel für die direkte Messung des Blutdrucks und für die intravenöse Verabreichung des Angiotensins I und der erfindungsgemässen Verbindungen versehen. Nach der Stabilisierung des basalen Blutdrucks wird die Druckänderung nach drei, in 5minutigen Intervallen durchgeführten, Reizungen mit 0,33 μg/kg Angiotensin I intravenös bestimmt. Druckänderungen werden auch 5, 10, 15, 30 und 60 min nach entweder intravenöser oder peroraler Verabreichung (mit Magensonde) der zu prüfenden Verbindungen bestimmt. Die letztgenannten Druckänderungen werden mit den Anfangswerten verglichen. Jede festgestellte Abnahme vom Ansprechen des Blutdrucks ist ein Hinweis auf die Hemmung des Enzyms, welches das Angiotensin umwandelt. Diese Abnahme kann bis zu 80%, nach Dosen von 10 mg/kg intravenös oder 50 mg/kg *p.o.*, oder niedrigen Dosen, reichen und bis 60 min aufrechterhalten bleiben.

Es ist illustrativ für die Erfindung, dass die 1-[4-(Pivaloyloxymethoxycarbonyl)-2R,4R-dimethylbutanoyl]indolin-2S-carbonsäuren und die 1-[4-(ℓ-Bornyloxycarbonylmethoxycarbonyl)-2R,4R-dimethylbutanoyl]indolin-2S-carbonsäure die Blutdrucksteigerung nach Angiotensin-I-Verabreichung an den Hund bei einer oralen Dosis von 10 mg/kg hemmen. Die Angiotensin-I-Untersuchungen am Hund werden am wachen Tier gemäss den nach Harris *et al.* entwickelten Methoden („Eur. J. Pharmacol.", *51*, 345 [1978]) durchgeführt.

Die *in vitro*-Hemmung des Enzyms, welches das Angiotensin umwandelt, kann bei den Verbindungen dieser Erfindung analog zu „Biochim. Biophys. Acta", *293*, 451 (1973) nachgewiesen werden. Gemäss dieser Methode werden die genannten Verbindungen in ungefähr 1-mmol-Konzentrationen in Phosphatpuffer, welcher von aussen mit Eis gekühlt wird, aufgelöst. Zu diesen Lösungen gibt man zuerst verschiedene μl-Mengen von 1 mmolarem Histidylleucin in Phosphatpuffer und dann 100 μl von 5 mmolarem Hippurylhistidylleucin in Phosphatpuffer und 50 μl des Angiotensin-umwandelnden Enzyms. Dieses Enzym wird aus Lungen von erwachsenen männlichen Kaninchen in Trispuffer, der Kalium- und Magnesiumchlorid und auch Rohrzucker enthält, frisch hergestellt. Die genannten Lösungen werden 30 min bei 37° C inkubiert und die weitere Reaktion wird durch Hinzufügen von 0,75 ml einer 0,6-normalen wässerigen Natriumhydroxidlösung gestoppt. Dann gibt man bei Zimmertemperatur 100 μl o-Phthalaldehyd und nach 10 min 100 μl 6-normaler Chlorwasserstoffsäure dazu. Die Proben werden in einem Spektrophotometer bei 360 nm mit Wasser verglichen und es wird ihre optische Dichte bestimmt. Diese Werte werden durch einen Konversionsfaktor einer Standardkurve angepasst, wobei man die während der genannten Inkubation von 30 min gebildete Histidylleucinmenge in Nanomolen erhält. Die Ergebnisse werden, um den $IC_{50}$-Wert zu bestimmen, gegenüber den Wirkstoffkonzentrationen graphisch als Kurve dargestellt. Dieser Wert bedeutet diejenige Wirkstoffkonzentration, welche die Hälfte der Aktivität einer Kontrollprobe, die keinen Wirkstoff enthält, ergibt. Die genannten repräsentativen Verbindungen der vorliegenden Erfindung sind auch in diesem *in vitro*-Testsystem sehr wirksam.

Dementsprechend sind die Verbindungen der Erfindung wertvolle antihypertensive Mittel, insbesondere für die Herabsetzung von hohem Blutdruck (ohne Rücksicht auf die Ätiologie) und/oder bei Herzerkrankungen wie Herzinsuffizienz, und/oder anderen Zuständen, die mit Ödemen oder Ascites einhergehen, z. B. Leberzirrhose. Sie können auch als Zwischenprodukte zur Herstellung von anderen wertvollen Produkten, insbesondere von pharmakologisch wirksamen Präparaten eingesetzt werden.

Besonders hervorzuheben sind Verbindungen der Formel II

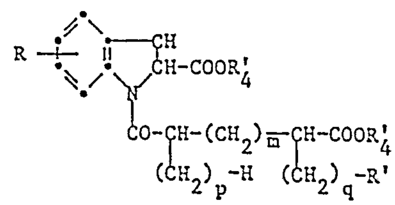
(II)

insbesondere ihre Indolin-2S-chiralen Epimere, worin R Wasserstoff, Alkyl oder Alkoxy mit höchstens je 4 Kohlenstoffatomen, Halogen oder Trifluormethyl bedeutet, m für die Zahl 0 oder 1 steht, jedes der Symbole p und q eine Zahl von 0 bis 2 bedeutet, und R' für Wasserstoff oder R-Phenyl steht, worin R-Phenyl unsubstituiertes oder durch Alkyl oder Alkoxy mit jeweils höchstens 4 Kohlenstoffatomen, Halogen oder Trifluormethyl substituiertes Phenyl bedeutet; eines der Symbole $R'_4$ für einen Substituenten der Gruppe Aryl(nieder)alkyl, worin Aryl Phenyl oder Pyridyl bedeutet, Bicycloalkyloxycarbonyl(nieder)alkyl, worin Bicycloalkyl Bornyl bedeutet oder Niederalkanoyloxy(nieder)alkyl steht, und das andere Symbol $R'_4$ Wasserstoff bedeutet, und ihre Salze, insbesondere ihre therapeutisch verwendbaren Salze, in erster Linie Alkalimetall- oder Ammoniumsalze der genannten Säuren.

Die Seitenkette

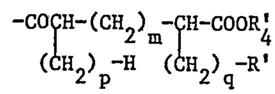

in den Verbindungen der Formel II kann in zwei verschiedenen diastereomeren Formen (hier erythro und threo genannt) existieren. Diese sind von der relativen Orientierung der Substituenten $(CH_2)_p-H$ und $(CH_2)_q-R'$ an der Kette (wenn keine dieser Gruppen Wasserstoff bedeutet) abhängig.

Bevorzugt sind Verbindungen der Formel II, welche die Seitenkette in der threo-Form haben, wobei die beiden Kohlenstoffatome, welche die genannten Substituenten tragen, vorzugsweise die (R)-Konfiguration aufweisen. Besonders bevorzugt sind die Verbindungen der weiter unten angegebenen Formel IIa, in welchen das Kohlenstoffatom in 2-Stellung des Indolins, welches die Carboxygruppe trägt, die (S)-Konfiguration hat, und eines oder beide Kohlenstoffatome, welche die Substituenten $(CH_2)_p-H$ und $(CH_2)_q-R'$ tragen, in der (R)-Konfiguration stehen (wenn die genannten Gruppen nicht Wasserstoff bedeuten).

Besonders hervorzuheben sind die Verbindungen der Erfindung, welche die Formel II haben,

worin R Wasserstoff, Methyl, Methoxy, Fluor, Chlor oder Trifluormethyl, vorzugsweise in 5-Stellung, bedeutet, jedes der Symbole m und p für 1 steht, q die Zahl 1 oder 2 bedeutet, R' für Wasserstoff oder Phenyl steht, und $R'_4$ die oben angegebene Bedeutung hat, und ihre Salze, insbesondere ihre therapeutisch anwendbaren Salze, in erster Linie Alkalimetall- oder Ammoniumsalze der genannten Säuren.

Die Verbindungen der vorliegenden Erfindung werden nach an sich bekannten Methoden vorzugsweise dadurch hergestellt, dass man

1) eine Verbindung der Formell III

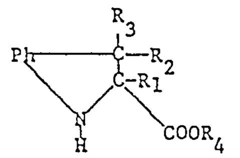

(III)

mit einem reaktionsfähigen funktionellen Derivat einer Verbindung der Formel IV

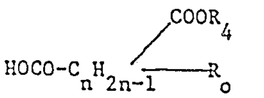

(IV)

in welchen Formel mindestens eines der Symbole $R_4$ sich vom Wasserstoff unterscheidet, kondensiert, oder

2) eine Verbindung der Formel V

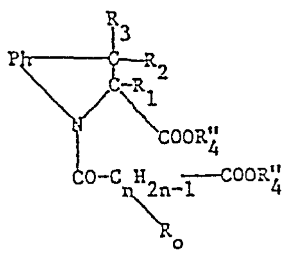

(V)

worin beide Symbole $R''_4$ Wasserstoff bedeuten oder eines Wasserstoff ist und das andere für einen Rest $R_4$ steht, verestert, und, wenn erwünscht, eine erhaltene Verbindung in eine andere Verbindung der Erfindung umwandelt.

Reaktionsfähige funktionelle Derivate von Verbindungen der Formel IV sind vorzugsweise Esterhalogenide oder Anhydride, z. B. die Halbester der genannten Säurechloride. Die genannte Kondensation von Verbindungen III und IV verläuft entweder spontan, oder in Gegenwart von Kondensationsmitteln, z. B. organischen oder anorganischen Basen, wie salzbildenden Aminen oder Metallcarbonaten, oder disubstituierten Carbodiimiden.

Die Veresterung einer Säure der Formel V wird nach an sich bekannten Methoden, z. B. durch Kondensation der genannten Disäure der Formel V oder eines ihrer Monoesterderivate mit einem Veresterungsmittel der Formel VI

$$R_4 - Z \qquad (VI)$$

worin Z Hydroxy oder vorzugsweise eine reaktionsfähige veresterte Hydroxygruppe bedeutet und $R_4$ für einen oben definierten Rest steht, hergestellt.

Eine reaktionsfähige veresterte Hydroxygruppe Z in einer Verbindung der Formel VI ist eine mit einer starken anorganischen oder organischen Säure veresterte Hydroxygruppe. Entsprechende Z-Gruppen sind insbesondere Halogen, z. B. Chlor, Brom oder vorzugsweise Jod, auch Sulfonyloxygruppen, wie Niederalkyl- oder Arylsulfonyloxygruppen, z. B. Methan-, Äthan-, Benzol- oder Toluolsulfonyloxygruppen.

Die Veresterung einer Carboxygruppe, ggf. in Salzform, mit einer Verbindung der Formel VI, worin Z eine reaktionsfähige veresterte Hydroxygruppe bedeutet, wird in an sich bekannter Weise vorgenommen. Man arbeitet in Gegenwart z. B. einer organischen Base, wie eines organischen Amins, z. B. eines tertiären Amins, wie Triniederalkylamins, z. B. Trimethylamin, Triäthylamin oder Äthyldiisopropylamin, eines N,N-Diniederalkylanilins, z. B. N,N-Dimethylanilin, eines cyclischen tertiären Amins, z. B. eines N-Niederalkylmorpholins, wie N-Methylmorpholin, einer Base vom Pyridintypus, z. B. Pyridin, einer anorganischen Base, z. B. von Hydroxiden, Carbonaten oder Hydrogencarbonaten von Alkalimetallen oder Erdalkalimetallen, z. B. Natrium-, Kalium- oder Calciumhydroxid, -carbonat oder -hydrogencarbonat, oder einer quaternären Ammoniumbase, z. B. eines Tetraalkylammoniumhydroxides, -carbonates oder -hydrogencarbonates, z. B. in welchem Alkyl z. B. Methyl, Äthyl, Propyl, Isopropyl, Butyl oder einen ähnlichen Rest bedeutet, oder eines Oxirans, z. B. eines 1,2-Niederalkylenoxids, wie Äthylenoxid oder Propylenoxid.

Die Dicarbonsäure der Formel V oder ihre Monoester wird vorzugsweise zuerst in ein Salz einer oben genannten organischen oder anorganischen Base, insbesondere in ihr Natrium- oder Kaliumsalz, umgewandelt und dann mit einer Verbindung VI umgesetzt. Die Verbindungen der Formel VI sind bekannt oder sie können nach an sich bekannten Methoden hergestellt werden.

Eine Verbindung der Formel VI, worin Z eine reaktionsfähige veresterte Hydroxygruppe bedeutet, kann unter den Reaktionsbedingungen hergestellt werden. So kann z. B. eine Verbindung der Formel VI, worin Z Chlor bedeutet, durch Behandlung mit Natriumjodid in einem Lösungsmittel, z. B. in Aceton oder Acetonitril, in eine Verbindung der Formel VI, worin Z für Jod steht, umgewandelt werden. Man kann aber die Veresterung auch mit einer Chlorverbindung der Formel VI in Gegenwart von Natriumjodid durchführen.

Die Veresterung wird in einem geeigneten inerten Lösungsmittel- oder Lösungsmittelgemisch, z. B. Dimethylformamid, in einem halogenierten Kohlenwasserstoff, z. B. Methylenchlorid, Tetrachlorkohlenstoff oder Chlorbenzol, in einem Keton, z. B. Aceton, einem Ester, z. B. Essigsäureäthylester, oder in einem Nitril, z. B. Acetonitril, oder in ihren Gemischen, vorgenommen. Man arbeitet vorzugsweise bei Raumtemperatur oder, wenn nötig, unter Kühlung oder bei erhöhter Temperatur, bei ungefähr −40 bis ungefähr 100, insbesondere bei −10 bis +40° C, und/oder in einer Inertgas-, z. B. Stickstoffatmosphäre.

Die Veresterung einer Carbonsäure mit einem Alkohol der Formel VI, worin Z Hydroxy bedeutet, wird nach an sich bekannten Methoden, vorzugsweise in Anwesenheit eines Säurekatalysators, z. B. Schwefelsäure oder Bortrifluoridätherat, insbesonder bei erhöhter Temperatur, vorzugsweise in einem Bereich von ungefähr 40 und 100° C, durchgeführt.

Die erhaltenen Verbindungen der Erfindung können in an sich bekannter Weise in andere Verbindungen der Erfindung übergeführt werden. So können z. B. erhaltene Diester durch selektive Hydrolyse, oder, wenn eine der Gruppen $R_4$ eine Benzylgruppe ist, durch Hydrogenolyse, z. B. mit Wasserstoff in Gegenwart eines Katalysators, z. B. Palladiumkatalysators, in Monoester umgewandelt werden. Erhaltene Ester können auch alkoholysiert (umestert) werden. Dies wird vorzugsweise durch Behandlung mit anorganischen Säuren, z. B. Halogenwasserstoff- oder Schwefelsäuren, in bekannter Weise, in Gegenwart eines der Formel $R_4-OH$ entsprechenden Alkohols, durchgeführt. Erhaltene freie Säuren können in an sich bekannter Weise in die genannten Metall- oder Ammoniumsalze umgewandelt werden.

So kann z. B. eine erhaltene freie Säure in ein entsprechendes Metall- oder Ammoniumsalz durch Umsetzung mit einer äquivalenten Menge einer entsprechenden Base, eines basischen Salzes oder eines Ionenaustauschers umgewandelt werden. Die genannten Säuren werden dabei mit Alkalimetall- oder Ammoniumhydroxiden oder -carbonaten behandelt. Ein erhaltenes Salz kann auch in die freie Verbindung, durch Behandlung mit stärkeren Säuren übergeführt werden.

Infolge der engen Beziehung zwischen den neuen Verbindungen in freier Form und in Form ihrer Salze sind im vorausgegangenen und nachfolgend unter freien Verbindungen (oder ihren Zwischenprodukten) und Salzen sinn- und zweckgemäss ggf. auch die entsprechenden Salze bzw. freien Verbindungen zu verstehen.

Die Ausgangsstoffe der Formeln III und IV sind bekannt oder können, falls sie neu sind, nach an sich bekannten Methoden, z. B. analog wie in den Beispielen beschrieben, hergestellt werden.

Ausgangsstoffe der Formel V können auch gemäss konventionellen Methoden erhalten werden.

Erhaltene geometrische oder optische Isomerengemische von obigen Verbindungen der Formeln I bis VI können nach an sich bekannten Methoden, z. B. durch fraktionierte Destillation, Kristallisation und/oder Chromatographie, in die einzelnen Isomeren getrennt werden. Racemische Produkte können in die optischen Antipoden, z. B. durch Trennung ihrer diastereomeren Salze, wie gemäss „J. Org. Chem.", *43*, 3803 (1978), z. B. durch fraktionierte Kristallisation von d- und ℓ-(Tartraten, Mandelaten, Camphersulfonaten oder 1-Naphthyl-1-äthylisocyanaten), oder von d- oder ℓ-(α-Methylbenzylammonium, Cinchonidin, Cinchonin, Chinin, Chinidin, Ephedrin, Dehydroabietylamin, Brucin oder Strychnin)salzen, gespalten werden. Der bevorzugte Ausgangsstoff der Formel III ist sein 2S-optisches Isomere (Epimere).

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, alkalischen oder sauren Kondensations- oder anderen, oben genannten Mitteln und/oder in einer inerten Atmosphäre, unter Kühlung, bei Zimmertemperatur oder bei erhöhten Temperaturen, vorzugsweise beim Siedepunkt des verwendeten Lösungsmittels, bei normalen oder erhöhtem Druck durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird.

Im Verfahren der vorliegenden Erfindung werden vorteilhafterweise solche Ausgangsstoffe verwendet, welche zu den im vorstehenden als besonders wertvoll beschriebenen Verbindungen, insbesondere solchen der Formel II, führen und die folgenden chiralen Isomeren sind

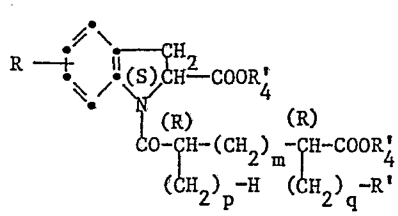

(IIa)

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z. B. Laktose, Dextrose, Rohrzucker, Mannitol, Sorbitol, Cellulose und/oder Glycin, und Schmiermitteln, z. B. Kieselerde, Talk, Sterainsäure oder Salze davon, wie Magnesium- oder Calciumstearat, und/oder Polyäthylenglykol, aufweisen; Tabletten enthalten ebenfalls Bindemittel, z. B. Magnesiumaluminiumsilikat, Stärkepaste, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, oder Brausemischungen, und/oder Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die

pharmakologischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere, pharmakologisch wertvolle Stoffe enthalten können, werden in an sich bekannter Weise, z. B. mittels konventioneller Misch-, Granulier- oder Dragierverfahren, hergestellt und enthalten von etwa 0,1 bis etwa 75, insbesondere von etwa 1 bis etwa 50% des Aktivstoffes. Einzeldosen für Säuger mit einem Gewicht von ungefähr 50-70 kg können zwischen ungefähr 5-100 mg des aktiven Bestandteils enthalten.

Die folgenden Beispiele dienen zur Illustration der Erfindung. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z. B. zwischen ungefähr 2 und 13 kPa (15 und 100 mmHg), durchgeführt.

*Beispiel 1:*

Man gibt zu 1,7 g 4-(Pivaloyloxymethoxycarbonyl)-2R,4R-dimethylbutanoylchlorid in 25 ml Pyridin bei Zimmertemperatur 1,2 g Indolin-2S-carbonsäurehydrochlorid. Das Reaktionsgemisch wird 3 h bei Zimmertemperatur gerührt und dann eingedampft. Der Rückstand wird in 75 ml gesättigter wässeriger Natriumhydrogencarbonatlösung gelöst und mit 2 × 50 ml Äther gewaschen. Die wässerige Schicht wird mit 10 ml 2-normaler wässeriger Chlorwasserstoffsäure angesäuert und mit 3 × 75 ml Methylenchlorid extrahiert. Die vereinigten Methylenchloridschichten werden über Magnesiumsulfat getrocknet und eingedampft. Man erhält die 1-[4-(Pivaloyloxymethoxycarbonyl)-2R,4R-dimethylbutanoyl]indolin-2S-carbonsäure. F. 109-112°. $[\alpha]_D = -136{,}2°$ (c = 0,94 in Äthanol), NMR ($CDCl_3$): 1,2, 1,8, 2,5, 3,35, 5,1, 5,75, 7,2, 8,3, 9,4 ppm. IR ($CHCl_3$): 3500-3200, 2950, 1740 $cm^{-1}$.

Der Ausgangsstoff wird wie folgt hergestellt: Ein Gemisch von 5,0 g 2R,4R-Dimethylglutarsäureanhydrid und 3,65 ml Benzylalkohol wird 3 h bei 85° gerührt. Man erhält die 4-(Carbobenzyloxy)-2R,4R-dimethylbutansäure. NMR ($CDCl_3$): 1,2, 1,8, 2,6, 5,2, 7,4, 11,2 ppm.

Die obige Säure (3,3 g) wird mit 5,9 ml 2,21-normaler Kaliumhydroxidlösung behandelt und die Lösung eingedampft. Der Rückstand wird mit Toluol (100 ml) versetzt und das Gemisch eingedampft. Man erhält das Kalium-4-(carbobenzyloxy)-2R,4R-dimethylbutanoat.

Man gibt zu Chlormethylpivalat (2,56 g) in 50 ml Aceton 2,54 g Natriumjodid. Das Reaktionsgemisch wird bei Raumtemperatur 3 h gerührt, filtriert und das Filtrat eingedampft. Der Rückstand wird in 25 ml Dimethylformamid aufgenommen und mit Kalium-4-(carbobenzyloxy)-2R,4R-dimethylbutanoat (3,7 g) in 25 ml Dimethylformamid versetzt. Das Reaktionsgemisch wird 18 h bei Raumtemperatur gerührt und dann eingedampft. Der Rückstand wird in 150 ml Äther gelöst, mit 3 × 50 ml 10%iger wässeriger Natriumhydrogencarbonatlösung und mit 3 × 50 ml gesättigter wässeriger Natriumchloridlösung gewaschen. Die organische Schicht wird über Magnesiumsulfat getrocknet und eingedampft. Man erhält das Pivaloyloxymethyl-4-(carbobenzyloxy)-2R,4R-dimethylbutanoat als ein Öl. NMR ($CDCl_3$): 1,2, 1,8, 2,5, 5,2, 5,8, 7,45 ppm.

Eine Lösung von 5,3 g Pivaloyloxymethyl-4-(carbobenzyloxy)-2R,4R-dimethylbutanoat in 150 ml Äthanol wird bei atmosphärischem Druck in Gegenwart von 0,5 g 10%igem Palladium-auf-Kohle-Katalysator hydriert. Das Reaktionsgemisch wird filtriert und eingedampft. Man erhält die 4-(Pivaloyloxymethoxycarbonyl)-2R,4R-dimethylbutansäure. NMR ($CDCl_3$): 1,2, 1,7, 2,55, 5,8 und 10,3 ppm.

Zu 1,7 g der obigen 4-(Pivaloyloxymethoxycarbonyl)-2R,4R-dimethylbutansäure in 30 ml Methylenchlorid gibt man bei Raumtemperatur 1,7 ml Oxalylchlorid. Das Reaktionsgemisch wird 2,5 h bei Raumtemperatur gerührt und dann eingedampft. Man erhält das 4-(Pivaloyloxymethoxycarbonyl)-2R,4R-dimethylbutanoylchlorid, welches in obiger Kondensation direkt verwendet wird. NMR ($CDCl_3$): 1,2, 1,9, 2,8, 5,85 ppm.

*Beispiel 2:*

Man gibt zu 6,5 g 4-(ℓ-Bornyloxycarbonylmethoxycarbonyl)-2R,4R-dimethylbutansäure in 100 ml Methylenchlorid 9,0 ml Oxalylchlorid. Das Reaktionsgemisch wird 3 h bei Raumtemperatur gerührt und dann eingedampft. Der Rückstand wird in 70 ml Pyridin gelöst und mit Indolin-2S-carbonsäurehydrochlorid (3,7 g) versetzt. Das Reaktionsgemisch wird 2,5 h bei Raumtemperatur gerührt und dann eingedampft. Der Rückstand wird in 75 ml gesättigter wässeriger Natriumhydrogencarbonatlösung gelöst und mit 3 × 50 ml Äther gewaschen. Die wässerige Schicht wird mit 10 ml 6-normaler Chlorwasserstoffsäure angesäuert und mit 3 × 75 ml Methylenchlorid extrahiert. Die vereinigten Methylenchloridextrakte werden über Magnesiumsulfat getrocknet und eingedampft. Man erhält das 1-[4-ℓ-Bornyloxycarbonylmethoxycabonyl)-2R,4R-dimethylbutanoyl]indolin-2S-carbonsäure. F. 65-67°. $[\alpha]_D = -139°$ (c = 0,915 in Äthanol). NMR ($CDCl_3$): 3,4, 4,9, 7,1-8,5 ppm. IR ($CHCl_3$): 3500-3100, 2950, 1735 $cm^{-1}$.

Der Ausgangsstoff wird wie folgt hergestellt: Ein Gemisch von 5,0 g ℓ-Borneol und 7,74 ml Chloracetylchlorid wird 3 h bei 100° gerührt und dann eingedampft. Man erhält das ℓ-Bornylchloracetat als ein Öl. NMR ($CDCl_3$): 0,88, 0,91, 1,1-2,5, 4,1, 5,0 ppm.

Man setzt zu 5,0 g des obigen ℓ-Bornylchloracetats in 70 ml Aceton 3,4 g Natriumjodid. Das Reaktionsgemisch wird 3 h bei Raumtemperatur gerührt, dann filtriert und eingedampft. Man erhält das ℓ-Bornyljodacetat, welches im nächsten Reaktionsschritt direkt verwendet wird.

Man vermischt ℓ-Bornyljodacetat (6,7 g) und Kalium-4-(carbobenzyloxy)-2R,4R-dimethylbu-

tanoat (6,1 g) in 100 ml Dimethylformamid. Das Reaktionsgemisch wird über Nacht bei Raumtemperatur gerührt und dann eingedampft. Der Rückstand wird in 200 ml Äther gelöst, mit 2 × 50 ml gesättigter wässeriger Natriumhydrogencarbonatlösung und mit 50 ml gesättigter wässeriger Natriumchloridlösung gewaschen. Die organische Schicht wird über Magnesiumsulfat getrocknet und eingedampft. Man erhält das ℓ-Bornyloxycarbonylmethyl-4-(carbobenzyloxy)-2R,4R-dimethylbutanoat. NMR ($CDCl_3$): 4,6, 5,1, 7,4 ppm.

Man hydriert das ℓ-Bornyloxycarbonylmethyl-4-(carbobenzyloxy)-2R,4R-dimethylbutanoat (8,0 g) in 125 ml Äthanol bei einem Druck von 1 atm in Gegenwart von 0,8 g 10%igem Palladium-auf-Kohle-Katalysator. Das Reaktionsgemisch wird filtriert und eingedampft. Man erhält die 4-(ℓ-Bornyloxycarbonylmethoxycarbonyl)-2R,4R-dimethylbutansäure. NMR ($CDCl_3$): 4,6, 5,0, 8,2 ppm.

*Beispiel 3:*

Man gibt bei 0° zu 1,1'-Carbonyldiimidazol (1,14 g) in 20 ml Methylenchlorid 4-(β-Methoxyäthoxymethoxycarbonyl)-2R,4R-dimethylbutansäure (1,6 g) in 20 ml Methylenchlorid. Nach 1 h gibt man tropfenweise innerhalb 10 min Indolin-2S-carbonsäurehydrochlorid (1,3 g) in 10 ml Pyridin dazu. Das Reaktionsgemisch wird über Nacht bei Raumtemperatur gerührt und dann eingedampft. Der Rückstand wird in 75 ml Methylenchlorid gelöst und mit 2 × 25 ml 2-normaler wässeriger Chlorwasserstoffsäure gewaschen. Die organische Schicht wird über Magnesiumsulfat getrocknet und eingedampft. Man erhält das Rohprodukt. Dieses wird in 20 ml Äther gelöst, zuerst mit 20 ml Essigsäureäthylester und 0,46 ml Dicyclohexylamin und dann mit 25 ml Pentan versetzt. Das Produkt wird abfiltriert. Man erhält das 1-[4-(β-Methoxyäthoxymethoxycarbonyl)-2R,4R-dimethylbutanoyl]indolin-2S-carbonsäuredicyclohexylammoniumsalz. F. 159-161°. $[\alpha]_D$ = −78,6° (c = 1,13 in Äthanol).

Der Ausgangsstoff wird wie folgt hergestellt: Man gibt zu 4 g 4-(Carbobenzyloxy)-2R,4R-dimethylbutansäure in 60 ml Äther, bei 0° 1,8 g Kaliumtert.-butoxid. Nach 30 min gibt man β-Methoxyäthoxymethylchlorid (1,82 g) dazu. Man lässt das Reaktionsgemisch innerhalb 1 h sich auf Raumtemperatur erwärmen und dann rührt man es weitere 2 h bei dieser Temperatur. Das Reaktionsgemisch wird in 50 ml gesättigte wässerige Natriumhydrogencarbonatlösung gegossen und mit 3 × 50 ml Äther extrahiert. Die vereinigten Ätherextrakte werden über Magnesiumsulfat getrocknet und eingedampft. Man erhält das β-Methoxyäthoxymethyl-4-(carbobenzyloxy)-2R,4R-dimethylbutanoat als ein Öl. $[\alpha]_D$ = −32,6° (c = 1,19 in Äthanol). NMR: 1,80, 3,35, 5,16, 5,30 ppm.

Eine Lösung des obigen Esters (0,5 g) in 25 ml Äthanol wird bei Raumtemperatur und einem Druck von 1 atm in Gegenwart von 50 mg 10%igem Palladium-auf-Kohle-Katalysator hydriert. Nach 2 h wird das Reaktionsgemisch abfiltriert und das Filtrat eingedampft. Man erhält die 4-(β-Methoxyäthoxymethoxycarbonyl)-2R,4R-dimethylbutansäure als ein Öl. $[\alpha]_D$ = −25,7° (c = 0,975 in Äthanol). NMR: 1,20, 1,83, 3,40, 5,40 ppm.

*Beispiel 4:*

Man versetzt 4-(3-Phthalidoxycarbonyl)-2R,4R-dimethylbutanoylchlorid (2,5 g) in 45 ml Pyridin mit Indolin-2S-carbonsäurehydrochlorid (1,3 g). Das Reaktionsgemisch wird bei Raumtemperatur 3 h gerührt und dann eingedampft. Der Rückstand wird in 100 ml Methylenchlorid mit 2 × 50 ml Wasser gewaschen. Die vereinigten wässerigen Teile werden mit konzentrierter wässeriger Chlorwasserstoffsäure auf den pH-Wert 1 eingestellt und mit 3 × 25 ml Methylenchlorid extrahiert. Die vereinigten Extrakte werden mit 2 × 25 ml 2-normaler wässeriger Chlorwasserstoffsäure gewaschen, über Magnesiumsulfat getrocknet und eingedampft. Man erhält einen Schaum, der aus Äther/Pentan umkristallisiert wird. Die erhaltenen 1-[4-(3-Phthalidoxycarbonyl)-2R,4R-dimethylbutanoyl]indolin-2S-carbonsäure schmilzt bei 119-122°. $[\alpha]_D$ = 104,9° (c = 0,65 in Äthanol).

Der Ausgangsstoff wird wie folgt hergestellt: Eine Lösung von 4-(Carbobenzyloxy)-2R,4R-dimethylbutansäure (0,3 g) in 6 ml 2-normaler wässeriger Kaliumhydroxidlösung wird zur Trokkene eingedampft. Der Rückstand wird zweimal mit 20 ml Toluol eingedampft. Der Rückstand wird in 50 ml Dimethylformamid aufgenommen und mit 3-Bromphthalid (2,55 g) versetzt. Das Reaktionsgemisch wird über Nacht bei Raumtemperatur gerührt und dann eingedampft. Der Rückstand wird in 40 ml Äther aufgenommen und mit 2 × 20 ml gesättigter wässeriger Natriumhydrogencarbonatlösung gewaschen. Die Ätherschicht wird über Magnesiumsulfat getrocknet und eingedampft. Man erhält den 4-(3-Phthalidoxycarbonyl)-2R,4R-dimethylbutansäurebenzylester als ein Öl. NMR ($CDCl_3$): 1,85, 2,60, 3,50, 5,15 ppm; IR (Film): 3035, 2978, 1775, 1755, 1730, 1468 $cm^{-1}$.

Der obige Ester (3,5 g) in 45 ml Äthanol wird bei einem Druck von 1 atm bei Zimmertemperatur in Gegenwart von 350 mg 10%igem Palladium-auf-Kohle-Katalysator hydriert. Nach 3 h wird das Reaktionsgemisch filtriert und das Filtrat eingedampft. Man erhält die 4-(3-Phthalidoxycarbonyl)-2R,4R-dimethylbutansäure. $[\alpha]_D$ = −21,3° (c = 0,78 in Äthanol). NMR: 1,2, 1,8, 2,65, 3,70 ppm.

Man versetzt 4-(3-Phthalidoxycarbonyl)-2R,4R-dimethylbutansäure (0,30 g) in 10 ml Methylenchlorid mit 0,24 ml Oxalylchlorid. Das Reaktionsgemisch wird bei Raumtemperatur 3 h gerührt und dann eingedampft. Man erhält das 4-(3-Phthalidoxycarbonyl)-2R,4R-dimethylbutanoylchlorid. $[\alpha]_D$ = −16,1° (c = 0,53 in Chloroform). NMR: 1,3, 1,9, 2,76, 4,3 ppm.

*Beispiel 5:*

Man gibt bei Raumtemperatur zu Indolin-2S-carbonsäurehydrochlorid (2,23 g) in 30 ml Pyridin

4-(Carbobenzyloxy)-2R,4R-dimethylbutanoylchlorid (3,3 g). Das Reaktionsgemisch wird 3 h bei Raumtemperatur gerührt und dann eingedampft. Der Rückstand wird in 100 ml gesättigter wässeriger Natriumhydrogencarbonaltlösung gelöst und mit 50 ml Äther gewaschen. Die wässerige Schicht wird mit konzentrierter Chlorwasserstoffsäure auf den pH-Wert 1 eingestellt und mit 3 × 50 ml Methylenchlorid extrahiert. Die vereinigten Methylenchloridextrakte werden über Florisil gerührt, über Magnesiumsulfat getrocknet und eingedampft. Man erhält die 1-[4-(Carbobenzyloxy)-2R,4R-dimethylbutanoyl]indolin-2S-carbonsäure. F. 150-153°. $[\alpha]_D = -139,1°$ (c = 0,79 in Äthanol).

Der Ausgangsstoff wird wie folgt hergestellt: Das obige Säurechlorid wird durch Rühren (3 h) von 3,0 g 4-(Carbobenzyloxy)-2R,4R-dimethylbutansäure und Oxalylchlorid (1,05 ml) in 50 ml Methylenchlorid bei Raumtemperatur und Abdampfen des Lösungsmittels hergestellt. Man erhält das 4-(Carbobenzyloxy)-2R,4R-dimethylbutanoylchlorid, welches in der obigen Kondensation direkt verwendet wird.

*Beispiel 6:*

Man gibt zum Säurechlorid, welches wie unten beschrieben, aus 2,5 g 2R,4R-Dimethylglutarsäureanhydrid hergestellt wird, in 40 ml Pyridin 3,2 g Indolin-2S-carbonsäurehydrochlorid. Das Reaktionsgemisch wird 3 h bei Raumtemperatur gerührt und dann eingedampft. Der Rückstand wird in 50 ml Wasser aufgenommen und mit 3 × 50 ml Methylenchlorid gewaschen. Die wässerige Schicht wird mit 2-normaler wässeriger Chlorwasserstoffsäure auf den pH-Wert 3 eingestellt und mit 3 × 50 ml Essigsäureäthylester extrahiert. Die vereinigten Extrakte werden über Magnesiumsulfat getrocknet und eingedampft. Der Rückstand wird mit 50 ml Äther trituriert. Man erhält die rohe 1-[4-(3-Pyridylmethoxycarbonyl)-2R,4R-dimethylbutanoyl] indolin-2S-carbonsäure. Diese wird in 30 ml 3-normaler wässeriger Chlorwasserstoffsäure gelöst und mit 30 ml Essigsäureäthylester gewaschen. Die wässerige Schicht wird eingedampft und der Rückstand bei 50° in Vakuum getrocknet. Man erhält das 1-[4-(3-Pyridylmethoxycarbonyl)-2R,4R-dimethylbutanoyl]indolin-2S-carbonsäurehydrochlorid. F. 86-89°. $[\alpha]_D = -75,2°$ (c = 0,8 in Äthanol).

Der Ausgangsstoff wird wie folgt hergestellt: Ein Gemisch von 2R,4R-Dimethylglutarsäureanhydrid (2,5 g) und 3-Pyridylcarbinol (1,7 ml) wird 6 h bei 90° gerührt und dann abgekühlt. Man erhält das rohe 4-(3-Pyridylmethoxycarbonyl)-2R,4R-dimethylbutansäure. NMR ($CDCl_3$: 8,83, 7,86, 7,44, 5,19, 2,58, 1,75, 1,18 ppm. IR (Film): 2980, 2935, 1735, 1465, 1163 $cm^{-1}$.

Die obige Säure in 50 ml Methylenchlorid wird mit 4,5 ml Oxalylchlorid 3 h bei Raumtemperatur gerührt. Nach Abdampfen des Lösungsmittels erhält man das Säurechlorid, welches ohne weitere Reinigung verwendet wird.

*Beispiel 7:*

Man gibt bei Raumtemperatur zu 1,9 g des Kaliumsalzes der 1-[4-(Carbobenzyloxy)-2R,4R-dimethylbutanoyl]indolin-2S-carbonsäure in 35 ml Dimethylformamid 1,15 g Jodmethylpivalat. Das Reaktionsgemisch wird über Nacht bei Raumtemperatur gerührt und dann eingedampft. Der Rückstand wird in 150 ml Äther aufgenommen, mit 50 ml gesättigter wässeriger Natriumhydrogencarbonatlösung und mit 50 ml gesättigter wässeriger Natriumchloridlösung gewaschen. Die organische Schicht wird über Magnesiumsulfat getrocknet und eingedampft. Man erhält das Pivaloyloxymethyl-1-[4-(carbobenzyloxy)-2R,4R-dithylbutanoyl]indolin-2S-carboxylat. NMR ($CDCl_3$): 1,20, 3,20, 5,70, 7,25 ppm. $R_f$ = 0,8 (90:9:1, Chloroform/Äthanol/Essigsäure. Silicagel).

*Beispiel 8:*

Eine Lösung von 0,60 g Pivaloyloxymethyl-1-[4-(carbobenzyloxy)-2R,4R-dimethylbutanoyl]-indolin-2S-carboxylat in 30 ml Äthanol wird bei Raumtemperatur, unter einem Druck von 1 atm in Gegenwart von 50 mg 10%igem Palladium-auf-Kohle-Katalysator hydriert. Das Reaktionsgemisch wird filtriert und das Filtrat eingedampft. Man erhält das Pivaloyloxymethyl-1-[4-carboxy-2R,4R-dimethylbutanoyl]indolin-2S-carboxylat. F. 143-145°. $[\alpha]_D = -139,6°$ (c = 0,9 in Äthanol).

*Beispiel 9:*

Man gibt bei 0° zu einer Lösung von 4-[1-(Äthoxycarbonyloxy)äthoxycarbonyl]-2R,4R-dimethylbutansäure (5,65 g) in 100 ml Methylenchlorid 1,1'-Carbonyldiimidazol (3,3 g). Nach 1 h gibt man Indolin-2S-carbonsäurehydrochlorid (4,1 g) und Triäthylamin (4,1 g) dazu und rührt das Reaktionsgemisch über Nacht bei Raumtemperatur. Das Gemisch wird mit 100 ml Methylenchlorid verdünnt und mit 50 ml eiskaltem Wasser, welches 4 ml 12-normaler Chlorwasserstoffsäure enthält, gewaschen. Die wässerige Phase wird mit 2 × 50 ml Methylenchlorid gewaschen. Die vereinigten Methylenchloridmengen werden über Magnesiumsulfat getrocknet und eingedampft. Man erhält die 1-{4-[1-Äthoxycarbonyloxy)äthoxycarbonyl]-2R,4R-dimethylbutanoyl}indolin-2S-carbonsäure.

Der Ausgangsstoff wird wie folgt hergestellt: Eine Lösung von 1-Chloräthyläthylcarbonat (5,50 g) und Natriumjodid (5,95 g) in 75 ml Aceton wird 1 h unter Rückfluss gekocht. Das Aceton wird im Vakuum abgedampft und der Rückstand zwischen 100 ml Äther und 100 ml Wasser verteilt. Die ätherische Schicht wird mit 25 ml 5%iger Natriummetabisulfitlösung gewaschen und über Magnesiumsulfat getrocknet. Der Äther wird im Vakuum abgedampft. Das zurückgebliebene Äthyl-1-jodäthylcarbonat wird gleich in 15 ml Dimethylformamid gelöst und zu einer Lösung von Kalium-4-(carbobenzyloxy)-2R,4R-dimethylbutanoat (10,4 g) in 50 ml Dimethylformamid gegeben. Das Reaktionsgemisch wird bei Zimmertemperatur 18 h gerührt und dann eingedampft. Der Rückstand wird in 150 ml Äther gelöst und mit 3 × 50 ml einer 10%igen wässerigen Natriumhydrogencarbonatlösung gewaschen. Die Ätherschicht wird über Magnesiumsulfat getrocknet und eingedampft. Man erhält das 1-(Äthoxycarbonyloxy)äthyl-4-(carbobenzyloxy)-2R,4R-dimethylbutanoat.

Eine Lösung des obigen Benzylesters (7,9 g) in 150 ml Äthanol wird unter atmosphärischem Druck in Gegenwart von 0,5 g 10%igem Palladium-auf-Kohle-Katalysator hydriert. Das Reaktionsgemisch wird filtriert und eingedampft. Man erhält die 4-[1-(Äthoxycarbonyloxy)äthoxycarbonyl]-2R,4R-dimethylbutansäure.

*Beispiel 10:*

Eine Lösung von 2,0 g ℓ-Bornyljodacetat und 2,30 g des Kaliumsalzes des 1-(4-Carboxy-2R,4R-dimethylbutanoyl)indolin-2S-carbonsäureäthylesters in 30 ml Dimethylformamid wird über Nacht bei Raumtemperatur gerührt und dann eingedampft. Der Rückstand wird in 100 ml Äther gelöst und mit 3 × 25 ml Wasser gewaschen. Die organische Schicht wird über Magnesiumsulfat getrocknet und eingedampft. Man erhält den 1-[4-(ℓ-Bornyloxycarbonylmethoxycarbonyl)-2R,4R-dimethylbutanoyl]indolin-2S-carbonsäureäthylester.

*Beispiel 11:*

Die folgenden Verbindungen werden gemäss den in den vorhergehenden Beispielen beschriebenen Verfahren hergestellt:

a) 1-(4-Carboxy-2R,4R-dimethylbutanoyl)-indolin-2S-carbonsäure-(1-äthoxycarbonyloxyäthyl)ester;

b) 1-(4-Carboxy-2R,4R-dimethylbutanoyl)-indolin-2S-carbonsäure-(ℓ-bornyloxycarbonylmethyl)ester;

c) 1-(4-Carboxy-4R-phenyläthylbutanoyl)-indolin-2S-carbonsäurepivaloyloxymethylester;

d) 1-[4-(Pivaloyloxymethoxycarbonyl)-2R-methyl-4R-phenyläthylbutanoyl]indolin-2S-carbonsäure;

e) 1-[4-(ℓ-Bornyloxycarbonylmethoxycarbonyl)-2R-methyl-4R-phenyläthylbutanoyl]-indolin-2S-carbonsäure.

*Beispiel 12:*

Herstellung von 10 000 Kapseln mit einem Gehalt von je 10 mg der aktiven Substanz:

*Bestandteile:*

| | |
|---|---|
| 1-[4-(Pivaloyloxymethoxycarbonyl)-2R,4R-dimethylbutanoyl]indolin-2S-carbonsäure | 100 g |
| Milchzucker | 1800 g |
| Talkpulver | 100 g |

*Verfahren:*

Sämtliche pulverigen Bestandteile werden mit einem Sieb von 0,6 mm Maschenweite gesiebt. Dann wird der Wirkstoff zuerst mit Talk und darauffolgend mit Milchzucker in einem geeigneten Mischer homogenisiert. Kapseln Nr. 3 werden mit je 200 mg des Gemisches mit einer Füllmaschine gefüllt.

In analoger Weise werden pharmazeutische Präparate von anderen erfindungsgemässen Verbindungen, z. B. solchen, die in den weiteren Beispielen hier illustriert sind, hergestellt.

*Kardiovaskuläre Pharmakologie von Verbindungen der Erfindung*

Die Prüfung der Wirkung von Verbindungen ist gemäss den Methoden für die Auswertung der Hemmung des Enzyms, welches das Angiotensin umwandelt (Angiotensin Converting Enzyme = ACE), durchgeführt worden. In der biochemischen Beurteilung der *in vitro*-ACE-Hemmung (ACE Inhibition = ACEI) wird die peptidolytische Aktivität einer Verbindung im Kaninchenlungengewebe bestimmt.

In der *in vivo*-Prüfungsmethode wird zuerst durch Verabreichung des Angiotensins I (AI) an das Versuchstier eine Blutdrucksteigerung hervorgerufen. Es wird dann die Hemmung der einzelnen Verbindungen auf diese Blutdrucksteigerung gemessen.

I. *Biochemische Testmethode*

Es wurde ein Kaninchenlungengewebepräparat (Das and Saffer, „J. Biol. Chem.", *250,* 6762 [1975]) für die Bestimmung von ACE nach der Methode von Cheung und Cushman (Cheung and Cushman, „Biochim. Biophys. Acta", *293,* 451 [1973]) verwendet. In dieser Methode wird die Menge des Histidylleucins, welche aus einem synthetischen Substrat innerhalb einer Inkubation von 30 min bei 37° freigesetzt wird, spektrophotometrisch bestimmt. Die $IC_{50}$-Werte der ACE-Hemmung werden dann graphisch berechnet. Der $IC_{50}$-Wert ist die Konzentration (in Mol) der getesteten Verbindung, welche die in Abwesenheit der Testsubstanz entstehende Menge des Histidylleucins um 50% herabsetzt.

II. *Methode der Hemmung der durch Angiotensin I (AI) hervorgerufenen Blutdrucksteigerung, durch intravenöse Verabreichung der Testverbindung (% AI-Hemmung)*

In diesen Untersuchungen wird, wie oben beschrieben, je ein Katheter in eine femurale Arterie und in eine Wadenvene der Ratte eingeführt. Der arterielle Druck wurde von dem Arterienkatheter aus registriert, während man Angiotensin I (AI) und die einzelnen Testverbindungen durch den Venenkatheter injiziert hatte. Die Hemmung der durch Angiotensin I verursachten Blutdrucksteigerung ist in der Tabelle als Durchschnittswert 30 min nach der Verabreichung von ausgewählten Dosen der jeweiligen Verbindung wiedergegeben. Dabei werden Dosierungen so ausgewählt, dass u. a. der $ID_{50}$(intravenös)-Wert ermittelt werden kann.

*Ergebnisse*

| Verbindung des Beispiels | *In vitro*-ACEI $IC_{50}$ (mol) | Hemmung der durch Angiotensin (AI) verursachten Blutdrucksteigerung | |
|---|---|---|---|
| | | i.v. Dosis (mg/kg) | AI-Hemmungen (%) |
| 1 | $3\times10^{-7}$ | 1,0 | 83 |
| 2 | $1\times10^{-5}$ | 1,0 | 75 |
| 3 | $3\times10^{-7}$ | 1,0 | 87 |
| 4 | $2\times10^{-7}$ | 1,0 | 89 |
| 5 | $3\times10^{-6}$ | 1,0 | 96 |
| 6 | $2\times10^{-6}$ | 1,0 | 90 |
| 8 | $2\times10^{-7}$ | 1,0 | 88 |

**Patentansprüche** für die Vertragsstaaten BE, CH, DE, FR, GB, IT, LI, LU, NL, SE

1. Verbindung der allgemeinen Formel (I)

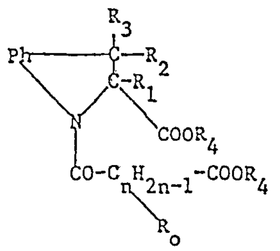
(I)

worin Ph unsubstituiertes 1,2-Phenylen oder durch einen bis drei, gleiche oder verschiedene Substituenten der Gruppe Niederalkyl, Niederalkoxy, Niederalkylendioxy, Hydroxy, Halogen oder Trifluormethyl substituiertes 1,2-Phenylen bedeutet, $R_o$ für Wasserstoff oder HPh steht, jedes der Symbole $R_1$, $R_2$ und $R_3$ Wasserstoff oder Niederalkyl bedeutet, n für eine ganze Zahl von 1 bis 10 steht; jedes der Symbole $R_4$ ist ein Substituent der Gruppe Aryl(nieder)alkyl, worin Aryl ein ggf. durch Niederalkyl, Niederalkoxy oder Halogen substituiertes Phenyl, oder Pyridyl bedeutet, Niederalkanoyloxy(nieder)alkyl, ggf. durch Niederalkyl, Niederalkoxy oder Halogen substituiertes Phthalidyl, (Hydroxy-, Niederalkanoyloxy- oder Niederalkoxy-)substituiertes (Nieder)alkoxymethyl, Bicycloalkyloxycarbonyl(nieder)alkyl, welches in der Bicycloalklygruppe höchstens 10 Kohlenstoffatome aufweist und ggf. durch Niederalkyl substituiert ist, oder 1-(Niederalkoxycarbonyloxy)niederalkyl; oder eines der Symbole $R_4$ für Wasserstoff steht und das andere einen oben genannten Rest bedeutet, wobei mit nieder bezeichnete Reste 1 bis 7 Kohlenstoffatome aufweisen, und ihre Salze.

2. Verbindungen der im Anspruch 1 gezeigten Formel (I), worin jedes der Symbole $R_4$ ein Substituent der Gruppe Aryl(nieder)alkyl ist, worin Aryl ein ggf. durch Niederalkyl, Niederalkoxy oder Halogen substituiertes Phenyl, oder Pyridyl bedeutet, Niederalkanoyloxy(nieder)alkyl, ggf. durch Niederalkyl, Niederalkoxy oder Halogen substituiertes Phthalidyl, (Hydroxy-, Niederalkanoyloxy- oder Niederalkoxy-)substituiertes (Nieder)alkoxymethyl, oder Bicycloalkyloxycarbonyl(nieder)alkyl, welches in der Bicycloalkylgruppe höchstens 10 Kohlenstoffatome aufweist und ggf. durch Niederalkyl substituiert ist; oder eines der Symbole $R_4$ für Wasserstoff steht und das andere einen oben genannten Rest bedeutet, und die anderen Symbole die im Anspruch 1 angegebenen Bedeutungen haben, wobei mit nieder bezeichnete Reste 1 bis 7 Kohlenstoffatome aufweisen, und ihre Salze.

3. Verbindungen der im Anspruch 1 gezeigten Formel (I), worin Ph unsubstituiertes 1,2-Phenylen, oder durch Niederalkyl, Niederalkoxy, Niederalkylendioxy, Hydroxy, Halogen oder Trifluormethyl monosubstituiertes 1,2-Phenylen bedeutet, $R_o$ für Wasserstoff oder HPh steht, jedes der Symbole $R_1$, $R_2$ und $R_3$ Wasserstoff oder Niederalkyl bedeutet, n für die ganze Zahl von 2 bis 8 steht, und $R_4$ die im Anspruch 1 angegebene Bedeutung hat, wobei mit nieder bezeichnete Reste 1 bis 7 Kohlenstoffatome aufweisen, und ihre Salze.

4. Verbindungen der im Anspruch 1 gezeigten Formel (I), worin $R_4$ die im Anspruch 2 angegebene Bedeutung hat, und die anderen Symbole die im Anspruch 3 angegebenen Bedeutungen haben.

5. Verbindungen der Formel (II)

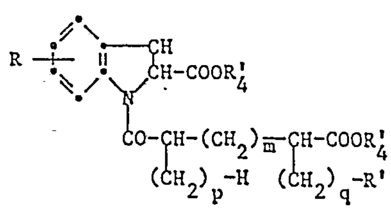
(II)

worin R Wasserstoff, Alkyl oder Alkoxy mit höchstens je 4 Kohlenstoffatomen, Halogen oder Trifluormethyl bedeutet, m für die Zahl 0 oder 1 steht, jedes der Symbole p und q eine Zahl von 0 bis 2 bedeutet, und R' Wasserstoff oder R-Phenyl steht, worin R-Phenyl unsubstituiertes oder durch Alkyl oder Alkoxy mit jeweils höchstens 4 Kohlenstoffatomen, Halogen oder Trifluormethyl substituiertes Phenyl bedeutet; eines der Symbole $R'_4$ für einen Substituenten der Gruppe Aryl(nieder)alkyl, worin Aryl Phenyl oder Pyridyl bedeutet, Bicycloalkyloxycarbonyl(nieder)alkyl, worin Bicycloalkyl Bornyl bedeutet oder Niederalkanyloxy(nieder)alkyl steht, und das andere Symbol $R'_4$ Wasserstoff bedeutet, wobei mit nieder bezeichnete Reste 1 bis 7 Kohlenstoffatome aufweisen, und ihre Salze.

6. Verbindungen der im Anspruch 5 gezeigten Formel (II), worin R Wasserstoff, Methyl, Methoxy, Fluor, Chlor oder Trifluormethyl bedeutet, jedes der Symbole m und p für 1 steht, q die Zahl 1 oder 2 bedeutet, R' für Wasserstoff oder Phenyl

steht, und $R'_4$ die im Anspruch 5 angegebene Bedeutung hat, und ihre Salze.

7. 1-[4-(Pivaloyloxymethoxycarbonyl)-2R,4R-dimethylbutanoyl]indolin-2S-carbonsäure und ihre Salze.

8. 1-[4-(ℓ-Bornyloxycarbonylmethoxycarbonyl)-2R,4R-dimethylbutanoyl]indolin-2S-carbonsäure oder ihre Salze.

9. Pharmazeutische Präparate enthaltend eine Verbindung der Ansprüche 1 und 3, oder ihre Salze, zusammen mit einem pharmazeutischen Trägermaterial.

10. Pharmazeutische Präparate enthaltend eine Verbindung der Ansprüche 2 und 4 bis 8, oder ihre Salze, zusammen mit einem pharmazeutischen Trägermaterial.

11. Die im Anspruch 1 genannten Verbindungen zur Anwendung in einem Verfahren zur therapeutischen Behandlung des menschlichen oder tierischen Körpers.

12. Die im Anspruch 2 genannten Verbindungen zur Anwendung in einem Verfahren zur therapeutischen Behandlung des menschlichen oder tierischen Körpers.

13. Die im Anspruch 1 genannten Verbindungen zur Anwendung als hypotensive, antihypertensive oder kardioaktive Mittel.

14. Die im Anspruch 2 genannten Verbindungen zur Anwendung als hypotensive, antihypertensive oder kardioaktive Mittel.

15. Verwendung der im Anspruch 1 genannten Verbindungen zur Herstellung von pharmazeutischen Präparaten.

16. Verwendung der im Anspruch 2 genannten Verbindungen zur Herstellung von pharmazeutischen Präparaten.

17. Verfahren zur Herstellung von den im Anspruch 1 genannten Verbindungen der Formel (I) und ihren Salzen, dadurch gekennzeichnet, dass man

1) eine Verbindung der Formel (III)

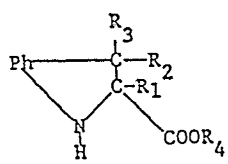

(III)

mit einem reaktionsfähigen funktionellen Derivat einer Verbindung der Formel (IV)

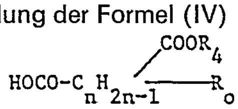

(IV)

in welchen Formeln mindestens eines der Symbole $R_4$ sich vom Wasserstoff unterscheidet, kondensiert, oder

2) eine Verbindung der Formel (V)

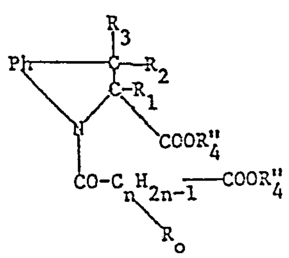

(V)

worin beide Symbole $R''_4$ Wasserstoff bedeuten oder eines Wasserstoff ist und das andere für einen Rest $R_4$ steht, verestert, und, wenn erwünscht, eine erhaltene Verbindung in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

18. Die nach dem Verfahren des Anspruchs 17 erhältlichen Verbindungen.

**Patentansprüche** für den Vertragsstaat: AT

1. Verfahren zur Herstellung von neuen 1-Carboxy(alkanoyl oder aralkanoyl)indolin-2-carbonsäureestern der allgemeinen Formel (I)

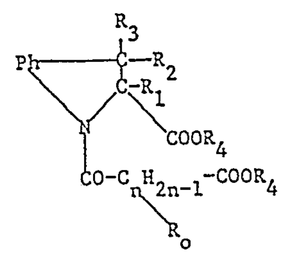

(I)

worin Ph unsubstituiertes 1,2-Phenylen oder durch einen bis drei, gleiche oder verschiedene Substituenten der Gruppe Niederalkyl, Niederalkoxy, Niederalkylendioxy, Hydroxy, Halogen oder Trifluormethyl substituiertes 1,2-Phenylen bedeutet, $R_o$ für Wasserstoff oder HPh steht, jedes der Symbole $R_1$, $R_2$ und $R_3$ Wasserstoff oder Niederalkyl bedeutet, n für eine ganze Zahl von 1 bis 10 steht; jedes der Symbole $R_4$ ist ein Substituent der Gruppe Aryl(nieder)alkyl, worin Aryl ein ggf. durch Niederalkyl, Niederalkoxy oder Halogen substituiertes Phenyl, oder Pyridyl bedeutet, Niederalkanoyloxy(nieder)alkyl, ggf. durch Niederalkyl, Niederalkoxy oder Halogen substituiertes Phthalidyl, (Hydroxy-, Niederalkanoyloxy- oder Niederalkoxy-)substituiertes (Nieder)alkoxymethyl, Bicycloalkyloxycarbonyl(nieder)alkyl, welches in der Bicycloalkylgruppe höchstens 10 Kohlenstoffatome aufweist und ggf. durch Niederalkyl substituiert ist, oder 1-(Niederalkoxycarbonyloxy)niederalkyl; oder eines der Symbole $R_4$ für Wasserstoff steht und das andere einen oben genannten Rest bedeutet, und ihren Salzen, wobei mit nieder bezeichnete Reste 1 bis 7 Kohlenstoffatome aufweisen, dadurch gekennzeichnet, dass man

1) eine Verbindung der Formel (III)

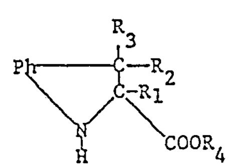

(III)

mit einem reaktionsfähigen funktionellen Derivat einer Verbindung der Formel (IV)

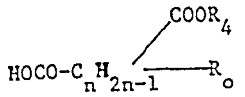

(IV)

in welchen Formeln mindestens eines der Symbole $R_4$ sich vom Wasserstoff unterscheidet, kondensiert, oder

2) eine Verbindung der Formel (V)

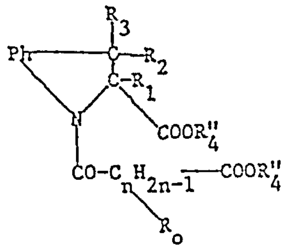

(V)

worin beide Symbole $R''_4$ Wasserstoff bedeuten oder eines Wasserstoff ist und das andere für einen Reste $R_4$ steht, verestert, und, wenn erwünscht, eine erhaltene Verbindung in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

2. Verfahren nach Anspruch 1, dadurch gekennzeichnet, dass man Verbindungen der im Anspruch 1 gezeigten Formel (I), worin jedes der Symbole $R_4$ ein Substituent der Gruppe Aryl(nieder)alkyl ist, worin Aryl ein ggf. durch Niederalkyl, Niederalkoxy oder Halogen substituiertes Phenyl oder Pyridyl bedeutet, Niederalkanoyloxy(nieder)alkyl, ggf. durch Niederalkyl, Niederalkoxy oder Halogen substituiertes Phthalidyl, (Hydroxy-, Niederalkanoyloxy- oder Niederalkoxy-)substituiertes (Nieder)alkoxymethyl, oder Bicycloalkyloxycarbonyl(nieder)alkyl, welches in der Bicycloalkylgruppe höchstens 10 Kohlenstoffatome aufweist und ggf. durch Niederalkyl substituiert ist; oder eines der Symbole $R_4$ für Wasserstoff steht und das andere einen oben genannten Rest bedeutet, und die anderen Symbole die im Anspruch 1 angegebenen Bedeutungen haben, und ihre Salze herstellt, wobei mit nieder bezeichnete Reste 1 bis 7 Kohlenstoffatome aufweisen.

3. Verfahren nach Anspruch 1, dadurch gekennzeichnet, dass man Verbindungen der im Anspruch 1 gezeigten Formel (I), worin Ph unsubstituiertes 1,2-Phenylen, oder durch Niederalkyl, Niederalkoxy, Niederalkylendioxy, Hydroxy, Halogen oder Trifluormethyl monosubstituiertes 1,2-Phenylen bedeutet, $R_o$ für Wasserstoff oder HPh steht, jedes der Symbole $R_1$, $R_2$ und $R_3$ Wasserstoff oder Niederalkyl bedeutet, n für die ganze Zahl von 2 bis 8 steht, und $R_4$ die im Anspruch 1 angegebene Bedeutung hat, und ihre Salze herstellt, wobei mit nieder bezeichnete Reste 1 bis 7 Kohlenstoffatome aufweisen.

4. Verfahren nach Anspruch 1, dadurch gekennzeichnet, dass man Verbindungen der im Anspruch 1 gezeigten Formel (I), worin $R_4$ die im Anspruch 2 angegebene Bedeutung hat, und die anderen Symbole die im Anspruch 3 angegebenen Bedeutungen haben, und ihre Salze, herstellt.

5. Verfahren nach Anspruch 1, dadurch gekennzeichnet, dass man Verbindungen der allgemeinen Formel (II)

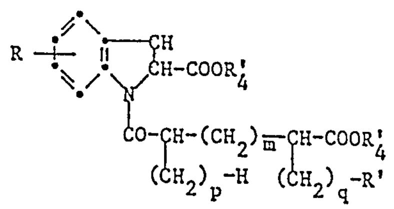

(II)

worin R Wasserstoff, Alkyl oder Alkoxy mit höchstens je 4 Kohlenstoffatomen, Halogen oder Trifluormethyl bedeutet, m für die Zahl 0 oder 1 steht, jedes der Symbole p und q eine Zahl von 0 bis 2 bedeutet, und R' für Wasserstoff oder R-Phenyl steht, worin R-Phenyl unsubstituiertes oder durch Alkyl oder Alkoxy mit jeweils höchstens 4 Kohlenstoffatomen, Halogen oder Trifluormethyl substituiertes Phenyl bedeutet; eines der Symbole $R'_4$ für einen Substituenten der Gruppe Aryl(nieder)alkyl, worin Aryl Phenyl oder Pyridyl bedeutet, Bicycloalkyloxycarbonyl(nieder)alkyl, worin Bicycloalkyl Bornyl bedeutet oder Niederalkanoyloxy(nieder)alkyl steht, und das andere Symbol $R'_4$ Wasserstoff bedeutet, und ihre Salze, herstellt, wobei mit nieder bezeichnete Reste 1 bis 7 Kohlenstoffatome aufweisen.

6. Verfahren nach Anspruch 1, dadurch gekennzeichnet, dass man Verbindungen der im Anspruch 5 gezeigten Formel (II), worin R Wasserstoff, Methyl, Methoxy, Fluor, Chlor oder Trifluormethyl bedeutet, jedes der Symbole m und p für 1 steht, q die Zahl 1 oder 2 bedeutet, R' für Wasserstoff oder Phenyl steht, und $R'_4$ die im Anspruch 5 angegebene Bedeutung hat, und ihre Salze, herstellt.

7. Verfahren nach Anspruch 1, dadurch gekennzeichnet, dass man 1-[4-(Pivaloyloxymethoxycarbonyl)-2R,4R-dimethylbutanoyl]indolin-2S-carbonsäure und ihre Salze herstellt.

8. Verfahren nach Anspruch 1, dadurch gekennzeichnet, dass man 1-[4-($\ell$-Bornyloxycarbonylmethoxycarbonyl)-2R,4R-dimethylbutanoyl]indolin-2S-carbonsäure und ihre Salze herstellt.

9. Verfahren zur Herstellung eines pharmazeutischen Präparates, gekennzeichnet durch die Verarbeitung eines erfindungsgemässen Wirkstoffes nach einem der Ansprüche 1 oder 3, mit einem pharmazeutischen Trägermaterial.

10. Verfahren zur Herstellung eines pharmazeutischen Präparates, gekennzeichnet durch die Verarbeitung eines erfindungsgemässen Wirkstoffes nach einem der Ansprüche 2 und 4 bis 8, mit einem pharmazeutischen Trägermaterial.

**Claims** for the Contracting States BE, CH, DE, FR, GB, IT, LI, LU, NL, SE

1. A compound of the general Formula (I)

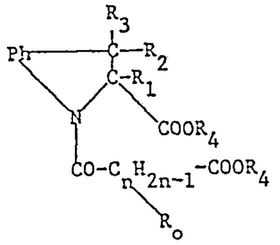

(I)

wherein Ph is unsubstituted 1,2-phenylene, or 1,2-phenylene substituted by one to three identical or different members selected from lower alkyl, lower alkoxy, lower alkylenedioxy, hydroxy, halogen and trifluoromethyl; $R_o$ is hydrogen or HPh; each of $R_1$, $R_2$ and $R_3$ is hydrogen or lower alkyl, n is an integer from 1 to 10, each of the symbols $R_4$ is a member selected from the group consisting of aryl-(lower) alkyl, in which aryl is unsubstituted phenyl or phenyl substituted by lower alkyl, lower alkoxy or halogen, or pyridyl, lower alkanoyloxy-(lower) alkyl, unsubstituted phthalidyl or phthalidyl substituted by lower alkyl, lower alkoxy or halogen, (hydroxy, lower alkanoyloxy or lower alkoxy)-substituted (lower) alkoxymethyl, bicycloalkyloxycarbonyl-(lower) alkyl, which contains not more than 10 carbon atoms in the bicycloalkyl group and is unsubstituted or substituted by lower alkyl, or 1-(lower alkoxycarbonyloxy)-lower alkyl; or one of the symbols $R_4$ is hydrogen and the other is a radical $R_4$ named above, with the radicals qualified by the term lower containing 1 to 7 carbon atoms, or a salt thereof.

2. A compound of the Formula (I) as claimed in Claim 1, wherein each of the symbols $R_4$ is a member selected from the group consisting of aryl-(lower) alkyl, in which aryl is unsubstituted phenyl or phenyl substituted by lower alkyl, lower alkoxy or halogen, or pyridyl lower alkanoyloxa-(lower) alkyl, unsubstituted phthalidyl or phthalidyl substituted by lower alkyl, lower alkoxy or halogen, (hydroxy, lower alkanoyloxy or lower alkoxy)-substituted (lower) alkoxymethyl, or bicycloalkyloxycarbonyl-(lower) alkyl which contains not more than 10 carbon atoms in the bicycloalkyl group and is unsubstituted or substituted by lower alkyl; or one of the symbols $R_4$ is hydrogen and the other is a radical $R_4$ named above, and the other symbols are as defined in Claim 1, with the radicals qualified by the term lower containing 1 to 7 carbon atoms, or a salt thereof.

3. A compound of the Formula (I) as claimed in Claim 1, wherein Ph is unsubstituted 1,2-phenylene, or 1,2-phenylene monosubstituted by lower alkyl, lower alkoxy, lower alkylenedioxy, hydroxy, halogen or trifluoromethyl; $R_o$ is hydrogen or HPh; each of $R_1$, $R_2$ and $R_3$ is hydrogen or lower alkyl; and n is an integer from 2 to 8; and $R_4$ is as defined in Claim 1, with the radicals qualified by the term lower containing 1 to 7 carbon atoms, or a salt thereof.

4. A compound of the Formula (I) as claimed in Claim 1, wherein $R_4$ is as defined in Claim 2, and the other symbols are as defined in Claim 3.

5. A compound of the Formula (II)

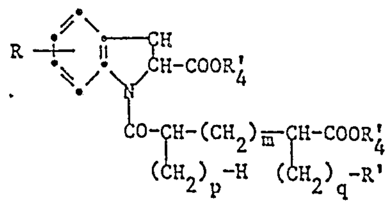

(II)

wherein R is hydrogen, alkyl or alkoxy, each containing not more than 4 carbon atoms, or halogen or trifluormethyl; m is the integer 0 or 1; each of p and q is an integer from 0 to 2; and R′ is hydrogen or R-phenyl, in which R-phenyl is unsubstituted phenyl or phenyl monosubstituted by alkyl or alkoxy, each containing not more than 4 carbon atoms, or by halogen or trifluoromethyl; one of the symbols $R'_4$ is a member selected from the group consisting of aryl-(lower) alkyl, in which aryl is phenyl or pyridyl, bicycloalkyloxycarbonyl-(lower) alkyl, in which bicycloalkyl is bornyl or lower alkanoyloxa-(lower) alkyl, and the other symbol $R'_4$ is hydrogen, with the radicals qualified by the term lower containing 1 to 7 carbon atoms, or a salt thereof.

6. A compound of the Formula (II) as claimed in Claim 5, wherein R is hydrogen, methyl, methoxy, fluorine, chlorine or trifluoromethyl, each of m and p is the integer 1, q is the integer 1 or 2, R′ is hydrogen or phenyl, and $R'_4$ is as defined in Claim 5, or a salt thereof.

7. 1-[4-(Pivaloyloxymethoxycarbonyl)-2R,4R-dimethylbutanoyl]indoline-2S-carboxylic acid, or a salt thereof.

8. 1-[4-(ℓ-Bornyloxycarbonylmethoxycarbonyl)-2R,4R-dimethylbutanolyl]indoline-2S-carboxylic acid, or a salt thereof.

9. A pharmaceutical composition containing a compound as claimed in Claims 1 and 3, or a salt thereof, together with a pharmaceutical carrier.

10. A pharmaceutical composition containing a compound as claimed in Claims 2 and 4 to 8, or a salt thereof, together with a pharmaceutical carrier.

11. Use of a compound as claimed in Claim 1 in a therapeutic method of treating the human or animal body.

12. Use of a compound as claimed in Claim 2 in a therapeutic method of treating the human or animal body.

13. Use of a compound as claimed in Claim 1 as hypotensive, antihypertensive or cardioactive agent.

14. Use of a compound as claimed in Claim 2 as hypotensive, antihypertensive or cardioactive agent.

15. Use of a compound as claimed in Claim 1 for the preparation of a pharmaceutical composition.

16. Use of a compound as claimed in Claim 2 for the preparation of a pharmaceutical composition.

17. A process for the preparation of a compound of Formula (I) as claimed in Claim 1, or of a salt thereof, which process comprises

(1) condensing a compound of Formula (III)

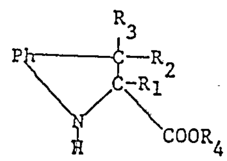

(III)

with a reactive functional derivative of a compound of Formula (IV)

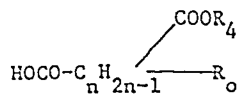

(IV)

in which formulae at least one of the symbols $R_4$ differs from hydrogen, or

(2) esterifying a compound of Formula (V)

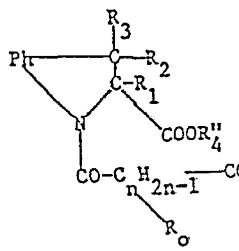

(V)

in which both of the symbols $R''_4$ are hydrogen or one is hydrogen and the other is a radical $R_4$, and, if desired, converting a resultant compound into another compound of this invention, and/or, if desired, converting a resultant free compound into a salt or a resultant salt into the free compound or into another salt, and/or, if desired, resolving a resultant mixture of isomers or racemates into the single isomers or racemates, and/or, if desired, resolving a resultant racemate into the optical antipodes.

18. A compound obtained by the process of Claim 17.

**Claims** for the Contracting State: AT

1. A process for the preparation of a novel 1-carboxy(alkanoyl or aralkanoyl)inoline-2-carboxylic acid ester of the general Formula (I)

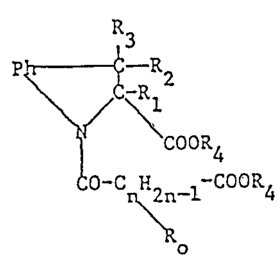

(I)

wherein Ph is unsubstituted 1,2-phenylene,or 1,2-phenylene substituted by one to three identical or different members selected from lower alkyl, lower alkoxy, lower alkylenedioxy, hydroxy, halogen and trifluoromethyl; $R_0$ is hydrogen or HPh; each of $R_1$, $R_2$ and $R_3$ is hydrogen or lower alkyl, n is an integer from 1 to 10, each of the symbols $R_4$ is a member selected from the group consisting of aryl-(lower) alkyl, in which aryl is unsubstituted phenyl or phenyl substituted by lower alkyl, lower alkoxy or halogen, or pyridyl, lower alkanoyloxi-(lower) alkyl, unsubstituted phthalidyl or phthalidyl substituted by lower alkyl, lower alkoxy or halogen, (hydroxy, lower alkanoyloxy or lower alkoxy)-substituted (lower) alkoxymethyl, bicycloalkyloxycarbonyl-(lower) alkyl, which contains not more than 10 carbon atoms in the bicycloalkyl group and is unsubstituted or substituted by lower alkyl, or 1-(lower alkoxycarbonyloxy)-lower alkyl; or one of the symbols $R_4$ is hydrogen and the other is a radical $R_4$ named above, or of a salt thereof, with the radicals qualified by the term lower containing 1 to 7 carbon atoms, which process comprises

(1) condensing a compound of Formula (III)

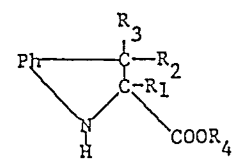

(III)

with a reactive functional derivative of a compound of Formula (IV)

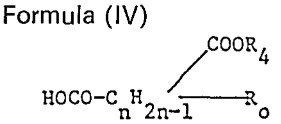

(IV)

in which formulae at least one of the symbols $R_4$ differs from hydrogen, or

(2) esterifying a compound of Formula (V)

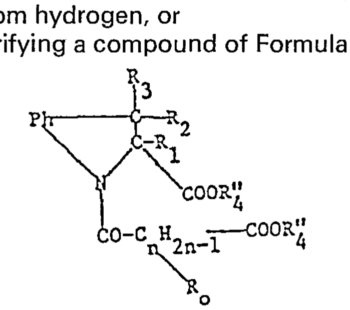

(V)

in which both of the symbols $R''_4$ are hydrogen or one is hydrogen and the other is a radical $R_4$, and, if desired, converting a resultant compound into another compound of this invention, and/or, if desired, converting a resultant free compound into a salt or a resultant salt into the free compound or into another salt, and/or, if desired, resolving a resultant mixture of isomers or racemates into the single isomers or racemates, and/or, if desired, resolving a resultant racemate into the optical antipodes.

2. A process according to Claim 1, which comprises preparing a compound of the Formula (I) as claimed in Claim 1, wherein each of the symbols $R_4$ is a member selected from the group consisting of aryl-(lower) alkyl, in which aryl is unsubstituted phenyl or phenyl substituted by lower alkyl, lower alkoxy or halogen, or pyridyl, lower alkanoyloxy-(lower) alkyl, unsubstituted phthalidyl or phthalidyl substituted by lower alkyl, lower alkoxy or halogen, (hydroxy, lower alkanoyloxy or lower alkoxy)-substituted (lower) alkoxymethyl, or bicycloalkyloxycarbonyl-(lower) alkyl which contains not more than 10 carbon atoms in the bicycloalkyl group and is unsubstituted or substituted by lower alkyl; or one of the symbols $R_4$ is hy-

drogen and the other is a radical $R_4$ named above, and the other symbols are as defined in Claim 1, or a salt thereof, with the radicals qualified by the term lower containing 1 to 7 carbon atoms.

3. A process according to Claim 1, which comprises preparing a compound of the Formula (I) as claimed in Claim 1, wherein Ph is unsubstituted 1,2-phenylene, or 1,2-phenylene monosubstituted by lower alkyl, lower alkoxy, lower alkylenedioxy, hydroxy, halogen or trifluoromethyl; $R_o$ is hydrogen or HPh; each of $R_1$, $R_2$ and $R_3$ is hydrogen or lower alkyl; and n is an integer from 2 to 8; and $R_4$ is as defined in Claim 1, or a salt thereof, with the radicals qualified by the term lower containing 1 to 7 carbon atoms.

4. A process according to Claim 1, which comprises preparing a compound of the Formula (I), wherein $R_4$ is as defined in Claim 2, and the other symbols are as defined in Claim 3, or a salt thereof.

5. A process according to Claim 1, which comprises preparing a compound of the general Formula (II)

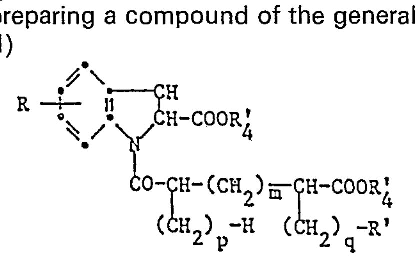

(II)

wherein R is hydrogen, alkyl or alkoxy, each containing not more than 4 carbon atoms, or halogen or trifluormethyl; m is the integer 0 or 1; each of p and q is an integer from 0 to 2; and R' is hydrogen or R-phenyl, in which R-phenyl is unsubstituted phenyl or phenyl monosubstituted by alkyl or alkoxy, each containing not more than 4 carbon atoms, or by halogen or trifluoromethyl; one of the symbols $R'_4$ is a member selected from the group consisting of aryl-(lower) alkyl, in which aryl is phenyl or pyridyl, bicycloalkyloxycarbonyl-(lower) alkyl, in which bicycloalkyl is bornyl or lower alkanoyloxy-(lower) alkyl, and the other symbol $R'_4$ is hydrogen, or a salt thereof, with the radicals qualified by the term lower containing 1 to 7 carbon atoms.

6. A process according to Claim 1, which comprises preparing a compound of the Formula (II) as claimed in Claim 5, wherein R is hydrogen, methyl, methoxy, fluorine, chlorine or trifluoromethyl, each of m and p is the integer 1, q is the integer 1 or 2, R' is hydrogen or phenyl, and $R'_4$ is as defined in Claim 5, or a salt thereof.

7. A process according to Claim 1, which comprises preparing 1-[4-(pivaloyloxymethoxycarbonyl)-2R,4R-dimethylbutanoyl]indoline-2S-carboxylic acid, or a salt thereof.

8. A process according to Claim 1, which comprises preparing 1-[4-(ℓ-bornyloxycarbonylmethoxycarbonyl)-2R,4R-dimethylbutanoyl]indoline-2S-carboxylic acid, or a salt thereof.

9. A process for the preparation of a pharmaceutical composition, which process comprises combining a compound of the invention as claimed in Claim 1 or 3 with a pharmaceutical carrier.

10. A process for the preparation of a pharmaceutical composition, which process comprises combining a compound of the invention as claimed in any one of Claims 2 and 4 to 8, with a pharmaceutical carrier.

**Revendications** pour les Etats contractants BE, CH, DE, FR, GB, IT, LI, LU, NL, SE

1. Composé de formule générale (I)

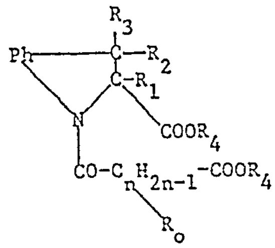

(I)

où Ph représente un 1,2-phénylène non substitué ou un 1,2-phénylène substitué par 1 à 3 substituants semblables ou différents du groupe alcoyle inférieur, alcoxy inférieur, alcoylène inférieur-dioxy, hydroxy, halogène ou trifluorométhyle, $R_o$ représente un hydrogène ou HPh, chacun des symboles $R_1$, $R_2$ et $R_3$ représente un hydrogène ou un alcoyle inférieur, n représente un nombre entier allant de 1 à 10; chacun des symboles $R_4$ est un substituant du groupe arylalcoyle(inférieur), où aryle représente un phényle éventuellement substitué par un alcoyle inférieur, un alcoxy inférieur ou un halogène, ou un pyridyle, un alcanoyloxy inférieur-alcoyle(inférieur), un phtalidyle éventuellement substitué par un alcoyle inférieur, un alcoxy inférieur ou un halogène, un alcoxyméthyle(inférieur) substitué par un hydroxy, alcanoyloxy inférieur ou alcoxy inférieur, un bicycloalcoyloxycarbonyl-alcoyle(inférieur), qui présente dans le groupe bicycloalcoyle au plus 10 atomes de carbone et est éventuellement substitué par un alcoyle inférieur, ou un 1-(alcoxycarbonyloxy inférieur)alcoyle inférieur; ou l'un des symboles $R_4$ représente un hydrogène et l'autre un radical mentionné ci-dessus, où les radicaux désignés par inférieurs présentent de 1 à 7 atomes de carbone, et leurs sels.

2. Composés de formule (I) présentée dans la revendication 1, où chacun des symboles $R_4$ est un substituant du groupe arylalcoyle(inférieur), où aryle représente un phényle éventuellement substitué par un alcoyle inférieur, un alcoxy inférieur ou un halogène, ou un pyridyle, un alcanoyloxy inférieur-alcoyle(inférieur), ou phtalidyle éventuellement substitué par un alcoyle inférieur, un alcoxy inférieur ou un halogène, un alcoxyméthyle(inférieur) substitué par un (hydroxy, alcanoyloxy inférieur ou alcoxy inférieur) ou un bicycloalcoyloxycarbonylalcoyle(inférieur), qui présente dans le groupe bicycloalcoyle au plus 10 atomes de carbone et est éventuellement substitué par un alcoyle inférieur; ou l'un des symboles $R_4$ représente un hydrogène et l'autre un radical mentionné cidessus, et les autres symboles ont les significations indiquées dans la revendication 1, où les radicaux

désignés par inférieurs présentent de 1 à 7 atomes de carbone, et leurs sels.

3. Composés de formule (I) présentée dans la revendication 1, où Ph représente un 1,2-phénylène non substitué, ou un 1,2-phénylène monosubstitué par un alcoyle inférieur, un alcoxy inférieur, un alcoylène inférieur-dioxy, un hydroxy, un halogène ou un trifluorométhyle, $R_0$ représente un hydrogène ou HPh, chacun des symboles $R_1$, $R_2$ et $R_3$ représente un hydrogène ou un alcoyle inférieur, n représente un nombre entier allant de 2 à 8, et $R_4$ a la signification indiquée dans la revendication 1.

4. Composés de formule (I) indiquée dans la revendication 1, où $R_4$ a la signification indiquée dans la revendication 2, et les autres symboles ont les significations indiquées dans la revendication 3.

5. Composés de formule (II)

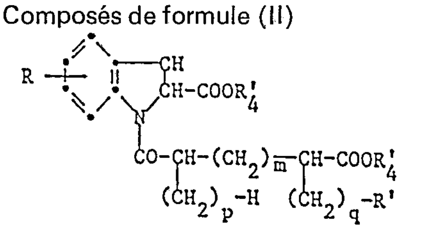

(II)

où R représente un hydrogène, un alcoyle ou un alcoxy ayant au plus chacun 4 atomes de carbone, un halogène ou un trifluorométhyle, m représente le nombre 0 ou 1, chacun des symboles p et q représente un nombre allant de 0 à 2, et R' représente un hydrogène ou un R-phényle, où R-phényle représente un phényle non substitué ou substitué par un alcoyle ou un alcoxy ayant à chaque fois au plus 4 atomes de carbone, un halogène ou un trifluorométhyle; l'un des symboles $R'_4$ représente un substituant du groupe arylalcoyle(inférieur), où aryle représente un phényle ou un pyridyle, un bicycloalcoyloxyalcoyle(inférieur), où bicycloalcoyle représente bornyle ou alcanoyloxy inférieuralcoyle(inférieur), et l'autre symbole $R'_4$ représente un hydrogène, où les radicaux designés par inférieurs présentent de 1 à 7 atomes de carbone, et leurs sels.

6. Composés de formule (II) présentée dans la revendication 2, où R représente un hydrogène, méthyle, méthoxy, fluor, chlore ou trifluorométhyle, chacun des symboles m et p vaut 1, q représente le nombre 1 ou 2, R' représente un hydrogène ou un phényle, et $R'_4$ a la signification indiquée dans la revendication 5, et leurs sels.

7. Acide 1-[4-(pivaloyloxyméthoxycarbonyl)-2R,4R-diméthylbutanoyl]indoline-2S-carboxylique et ses sels.

8. Acide 1-[4-(ℓ-bornyloxycarbonylméthoxycarbonyl)-2R,4R-diméthylbutanoyl]indoline-2S-carboxylique ou ses sels.

9. Préparations pharmaceutiques contenant un composé des revendications 1 et 3, ou ses sels, avec un support pharmaceutique.

10. Préparations pharmaceutiques contenant un composé des revendications 2 et 4 à 8, ou ses sels, avec un support pharmaceutique.

11. Les composés mentionnés dans la revendication 1 aux fins d'application dans un procédé de traitement thérapeutique du corps humain ou animal.

12. Les composés mentionnés dans la revendication 2 aux fins d'application dans un procédé de traitement thérapeutique du corps humain ou animal.

13. Les composés mentionnés dans la revendication 1 aux fins d'application comme agents hypotenseurs, antihypertenseurs ou cardioactifs.

14. Les composés mentionnés dans la revendication 2 aux fins d'application comme agents hypotenseurs, antihypertenseurs ou cardioactifs.

15. Application des composés mentionnés dans la revendication 1 à la préparation de préparations pharmaceutiques.

16. Application des composés mentionnés dans la revendication 2 à la préparation de préparations pharmaceutiques.

17. Procédé de préparation des composés de formule (I) mentionnés dans la revendication 1 et de leurs sels, caractérisé en ce que

1) on condense un composé de formule (III)

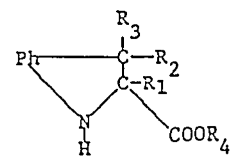

(III)

avec un dérivé fonctionnel réactif d'un composé de formule (IV)

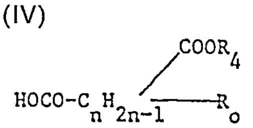

(IV)

formules dans lesquelles au moins l'un des symboles $R_4$ se différencie de l'hydrogène, ou

2) on estérifie un composé de formule (V)

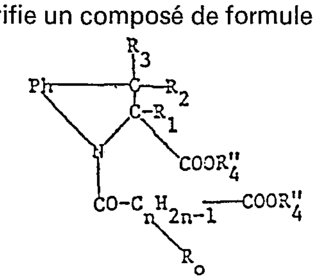

(V)

où les deux symboles $R''_4$ représentent un hydrogène ou l'un est un hydrogène et l'autre représente un radical $R_4$ et, si on le désire, on transforme un composé obtenu en un autre composé de l'invention et/ou, si on le désire, on transforme un composé libre obtenu en un sel ou un sel obtenu en le composé libre ou en un autre sel et/ou, si on le désire, on sépare un mélange obtenu d'isomères ou de racémates en les isomères ou racémates isolés et/ou, si on le désire, on dédouble les racémates obtenus en les antipodes optiques.

18. Les composés que l'on peut obtenir selon le procédé de la revendication 17.

**Revendications** pour l'Etat contractant: AT

1. Procédé de préparation de nouveaux esters d'acide 1-carboxy(alcanoyl ou aralcanoyl)indoline-2-carboxylique de formule générale (I)

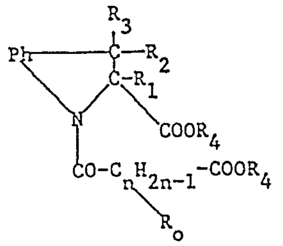

(I)

où Ph représente un 1,2-phénylène non substitué ou un 1,2-phénylène substitué par 1 à 3 substituants semblables ou différents du groupe alcoyle inférieur, alcoxy inférieur, alcoylène inférieur-dioxy, hydroxy, halogène ou trifluorométhyle, $R_o$ représente un hydrogène ou HPh, chacun des symboles $R_1$, $R_2$ et $R_3$ représente un hydrogène ou un alcoyle inférieur, n représente un nombre entier allant de 1 à 10; chacun des symboles $R_4$ est un substituant du groupe arylalcoyle(inférieur), où aryle représente un phényle éventuellement substitué par un alcoyle inférieur, un alcoxy inférieur ou un halogène, ou un pyridyle, un alcanoyloxy inférieur-alcoyle(inférieur), un phtalidyle éventuellement substitué par un alcoyle inférieur, un alcoxy inférieur ou un halogène, un alcoxyméthyl(inférieur) substitué par un hydroxy, alcanoyloxy inférieur ou alcoxy inférieur, un bicycloalcoyloxycarbonylalcoyle(inférieur), qui présente dans le groupe bicycloalcoyle au plus 10 atomes de carbone et est éventuellement substitué par un alcoyle inférieur, ou un 1-(alcoxycarbonyloxy inférieur)alcoyle inférieur; ou l'un des symboles $R_4$ représente un hydrogène et l'autre un radical mentionné ci-dessus, où les radicaux désignés par inférieurs présentent de 1 à 7 atomes de carbone, et leurs sels, caractérisé en ce que

1) on condense un composé de formule (III)

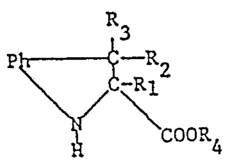

(III)

avec un dérivé fonctionnel réactif d'un composé de formule (IV)

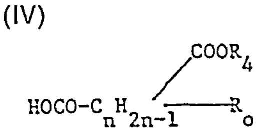

(IV)

formules dans lesquelles au moins l'un des symboles $R_4$ se différencie de l'hydrogène, ou

2) on estérifie un composé de formule (V)

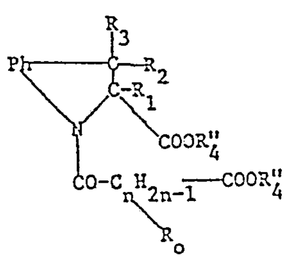

(V)

où les deux symboles $R''_4$ représentent un hydrogène ou l'un est un hydrogène et l'autre représente un radical $R_4$, et, si on le désire, on transforme un composé obtenu en un autre composé de l'invention et/ou, si on le désire, on transforme un composé libre obtenu en un sel ou un sel obtenu en le composé libre ou en un autre sel et/ou, si on le désire, on sépare un mélange obtenu d'isomères ou de racémates en les isomères ou racémates isolés et/ou, si on le désire, on dédouble les racémates obtenus en les antipodes optiques.

2. Procédé selon la revendication 1, caractérisé en ce qu'on prépare des composés de formule (I) présentée dans la revendication 1, où chacun des symboles $R_4$ est un substituant du groupe arylalcoyle(inférieur), où aryle représente un phényle éventuellement substitué par un alcoyle inférieur, un alcoxy inférieur ou un halogène, ou un pyridyle, un alcanoyloxy inférieur-alcoyle(inférieur), un phtalidyle éventuellement substitué par un alcoyle inférieur, un alcoxy inférieur ou un halogène, un alcoxyméthyle(inférieur) substitué par un (hydroxy, alcanoyloxy inférieur ou alcoxy inférieur) ou un bicycloalcoyloxycarbonylalcoyle(inférieur), qui présente dans le groupe bicycloalcoyle au plus 10 atomes de carbone et est éventuellement substitué par un alcoyle inférieur; ou l'un des symboles $R_4$ représente un hydrogène et l'autre un radical mentionné ci-dessus, et les autres symboles ont les significations indiquées dans la revendication 1, et leurs sels, où les radicaux désignés par inférieurs présentent de 1 à 7 atomes de carbone.

3. Procédé selon la revendication 1, caractérisé en ce qu'on prépare des composés de formule (I) présentée dans la revendication 1, où Ph représente un 1,2-phénylène non substitué, ou un 1,2-phénylène monosubstitué par un alcoyle inférieur, un alcoxy inférieur, un alcoylène inférieur-dioxy, un hydroxy, un halogène ou un trifluorométhyle, $R_o$ représente un hydrogène ou HPh, chacun des symboles $R_1$, $R_2$ et $R_3$ représente un hydrogène ou un alcoyle inférieur, n représente un nombre entier allant de 2 à 8, et $R_4$ a la signification indiquée dans la revendication 1, et leurs sels, où les radicaux désignés par inférieurs présentent de 1 à 7 atomes de carbone.

4. Procédé selon la revendication 1, caractérisé en ce qu'on prépare des composés de formule (I) présentée dans la revendication 1, où $R_4$ a la signification indiquée dans la revendication 2, et les autres symboles ont la signification indiquée dans la revendication 3, et leurs sels.

5. Procédé selon la revendication 1, caractérisé en ce qu'on prépare des composés de formule générale (II)

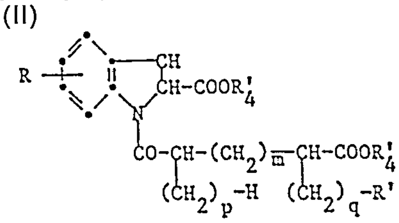

(II)

où R représente un hydrogène, un alcoyle ou un alcoxy ayant au plus chacun 4 atomes de carbone, un halogène ou un trifluorométhyle, m représente le nombre 0 ou 1, chacun des symboles p et q représente un nombre allant de 0 à 2, et R' représente un hydrogène ou un R-phényle, où R-phényle représente un phényle non substitué ou substitué par un alcoyle ou un alcoxy ayant à chaque fois au plus 4 atomes de carbone, un halogène ou un trifluorométhyle; l'un des symboles $R'_4$ représente un substituant du groupe arylalcoyle(inférieur), où aryle représente un phényle ou un pyridyle, un bicycloalcoyloxyalcoyle(inférieur), où bicycloalcoyle représente bornyle ou alcanoyloxy inférieur-alcoyle(inférieur), et l'autre symbole $R'_4$ représente un hydrogène, et leurs sels, où les radicaux désignés par inférieurs présentent de 1 à 7 atomes de carbone.

6. Procédé selon la revendication 1, caractérisé en ce qu'on prépare des composés de formule (II) présentée dans la revendication 5, où R représente un hydrogène, méthyle, méthoxy, fluor, chlore ou trifluorométhyle, chacun des symboles m et p vaut 1, q représente le nombre 1 ou 2, R' représente un hydrogène ou un phényle, et $R'_4$ a la signification donnée dans la revendication 5, et leurs sels.

7. Procédé selon la revendication 1, caractérisé en ce qu'on prépare l'acide 1-[4-(pivaloyloxyméthoxycarbonyl)-2R,4R-diméthylbutanoyl]indoline-2S-carboxylique et ses sels.

8. Procédé selon la revendication 1, caractérisé en ce qu'on prépare l'acide 1-[4-(ℓ-bornyloxycarbonylméthoxycarbonyl)-2R,4R-diméthylbutanoyl]indoline-2S-carboxylique et ses sels.

9. Procédé de préparation d'une préparation pharmaceutique, caractérisé par la transformation d'une substance active de l'invention selon l'une des revendications 1 ou 3, avec un support pharmaceutique.

10. Procédé de préparation d'une préparation pharmaceutique, caractérisé par la transformation d'une substance active de l'invention selon l'une des revendications 2 et 4 à 8, avec un support pharmaceutique.